# EUROPEAN PATENT APPLICATION

(11) **EP 2 305 274 A1**
(43) Date of publication of application: **06.04.2011**
(21) Application number: 09305882.4
(22) Date of filing: 21.09.2009
(51) Int. Cl.: A61K 33/14, A61K 31/404, A61P 25/28, A61K 45/00

(54) **Use of WNT stimulators for treating demyelinating diseases**

(71) Applicant: Centre National de la Recherche Scientifique (CNRS), 75016 Paris (FR); UNIVERSITE PARIS DESCARTES, 75006 Paris (FR)
(72) Inventor: The designation of the inventor has not yet been filed
(74) Representative: Warcoin, Jacques

(57) **Abstract**

The present invention relates to the use of an effective amount of a Wnt, a Wnt-like substance, and/or a compound stimulating the Wnt signalling pathway for the preparation of a pharmaceutical composition intended to reduce the extent or degree of nerve demyelination in the central and peripheric nervous system of an individual suffering from a demyelinating disorder.

## Description

### FIELD OF THE INVENTION

The present invention relates to treatments of demyelinating disorders. More specifically, the invention relates to the use of activators of Wnt signalling pathway for treating demyelinating conditions, especially Pelizaeus-Merzbacher disease (PMP) ,Charcot-Marie-Tooth disease and nerve injuries.

### BACKGROUND OF THE INVENTION

Myelination is a complex and fine-tuned process that involves multiple signaling pathways orchestrated by several cellular actors: the neurons and the Schwann cells (SC) which are the myelinating glial cells of the peripheral nervous system (PNS) and the oligodendrocytes, which are the myelinating glial cells of the central nervous system (CNS), respectively.

Demyelinating conditions are manifested in loss of myelin - the basis of the multiple dense layers of lipids and protein which cover many nerve fibers. In patients with demyelinating conditions, demyelination may be irreversible; it is usually accompanied or followed by axonal degeneration, and often by cellular degeneration. Demyelination can occur as a result of neuronal damage or damage to the myelin itself - whether due to aberrant immune responses, local injury, ischemia, metabolic disorders, toxic agents, or viral infections.

Central demyelination (demyelination of the CNS) occurs in several conditions, often of uncertain etiology, that have come to be known as the primary demyelinating diseases. Of these, multiple sclerosis is the most prevalent. Other primary demyelinating diseases include adrenoleukodystrophy, adrenomyeloneuropathy, AIDS-vacuolar myelopathy, HTLV-associated myelopathy, Leber's hereditary optic atrophy, progressive multifocal leukoencephalopathy, subacute sclerosing panencephalitis, and tropical spastic paraparesis. In addition, there are acute conditions in which demyelination can occur in the CNS, e.g., acute disseminated encephalomyelitis and acute viral encephalitis. Furthermore, acute transverse myelitis, a syndrome in which an acute spinal cord transection of unknown cause affects both gray and white matter in one or more adjacent thoracic segments, can also result in demyelination.

Current treatments for the various demyelinating conditions are expensive, symptomatic, and only partially effective, and may cause undesirable secondary effects. For example, corticosteroids (oral prednisone at 60-100 mg/day, tapered over 2-3 weeks, or intravenous methylprednisolone at 500-1000 mg/day, for 3-5 days), which represent the main form of therapy for MS can cause numerous medical complications, including osteoporosis, ulcers, and diabetes (Id.). Immunomodulatory therapy with recombinant human interferon-β (Bseron and Avonex) and with co-polymer (Copaxon) are currently used as treatment modalities for MS, but all are exceedingly expensive. Immunosuppressive drugs (azathioprine, cladribine, cyclophosphamide, and methotrexate) are used for more severe progressive forms. However, they are not uniformly beneficial, and have significant toxic side-effects. Several drugs (e.g., baclofen at 30-60 mg/day in divided doses) may reduce spasticity by inhibiting the spinal cord reflexes. Cautious and judicious use is required, though, because the drug-induced reduction in spasticity in MS patients often exacerbates weakness, thereby further incapacitating the patient. Similarly, current treatment for adrenoleukodystrophy, another devastating demyelinating disease, is relatively ineffective. Symptoms of adrenoleukodystrophy may include cortical blindness, corticospinal tract dysfunction, mental deterioration, and spasticity. Therapy to control the course of adrenoleukodystrophy may include bone marrow transplantation and dietary treatment, but inexorable neurological deterioration invariably occurs, ultimately leading to death. Some progress has been realized in the treatment of animals with EAE and experimental autoimmune neuritis, by using glial cell transplants and growth factors, and by inhibiting adhesion molecules, autoantibodies, and cytokines. However, none of these treatments has been shown to be beneficial in humans, and some require extensive neurosurgical intervention.

Peripheral and central myelin genes expression (Myelin protein zero (MPZ), Peripheral Myelin Protein 22 KDa (PMP22), Proteolipid Protein (PLP)) is tightly regulated in myelinating cells. As a matter of fact, in Schwann cells, slight changes of PMP22 or MPZ gene dosage have deep impact on the development and preservation of nerve. For example, either reduction or overexpression of PMP22 or MPZ can result in hereditary neuropathies like Charcot-Marie-Tooth disease (CMT1A or CMT1 B). In oligodendrocytes, the level of PLP gene is also strictly regulated as either overexpression or absence of PLP protein leads to severe diseases in rodents as well as humans (Pelizaeus-Merzbacher disease (PMD)). The expression of PMP22 and MPZ is stimulated during myelination process which occurs after birth between postnatal day 0 (P0) and 21 (P21). After that, those genes are expressed at low levels in order to maintain myelin turnover. They are re-expressed at higher levels only after nerve injuries to initiate remyelination. In the mice CNS, myelination starts at birth in the spinal cord and ends around 45-60 days postnatal in brain. The timing of myelination is very precise and depends on the brain region.

The Charcot-Marie-Tooth disease (CMT), also known as Hereditary Motor and Sensory Neuropathy (HMSN), Hereditary Sensorimotor Neuropathy (HSMN), or Peroneal Muscular Atrophy, is a heterogeneous inherited disorder of nerves (neuropathy) that is characterized by loss of muscle tissue and touch sensation, predominantly in the feet and legs but also in the hands and arms in the advanced stages of disease. It is mainly caused by the alteration of the myelin gens PMP22 or MPZ. Presently incurable, this disease is one of the most common inherited neurological disorders, with 37 in 100,000 affected (Krajewski KM et al, Brain 2000).

Pelizaeus-Merzbacher disease (PMD) is an X-linked developmental disorder affecting myelin formation in the nervous system and is caused by a mutation in the proteolipid protein gene (PLP) associated mainly with oligodendrocytes in the CNS.

Few cellular signals are able to regulate the expression of myelin genes. Steroid hormones like progesterone and glucocorticoid (Désarnaud F. et al., *Brain research* 2000) stimulate myelin genes expression either directly by means of their nuclear receptors or indirectly by enhancing the expression of Krox-20 and Sox-10 transcription factors which are essential for myelin gene expression (Magnaghi V et al, *J. Mol. Neuroscience* 2007).

Therefore, authors have suggested using antiprogesterone therapy (Mifepristone, Onapristone) to treat CMT1A pathology (Meyer zu Hörste G et al, *Ann. neurol.* 2007). Furthermore, ascorbic acid is able to inhibit PMP22 expression by reducing cAMP levels (Kaya F, *Neuromuscul disorder* 2007). Consequently, vitamin C was proposed as treatment of CMT1A based on inhibiting PMP22 gene dosage. PMP22 is also a target of microRNA-29a which is able to dampen its expression in a post-transcriptional manner (Verrier DA *Glia.* 2009).

Several other treatment have been proposed for enhancing myelination, for example calcium-channel blockers (US2009/006929) and a combination of transferring and insulin-growth factor I (IGF-1) (W02005112911), but a number of demyelinating diseases, in particular the leukodystrophies and the Charcot-Marie-Tooth disease (CMT) are still presently incurable.

Thus, it is clear from the foregoing that there exists a need for more effective methods to treat the varied array of demyelinating diseases.

In this context, the present inventors demonstrate here for the first time the importance of the Wnt pathway in the regulation of myelin gene expression. Indeed, they have shown that Wnt1 (a Wnt / β-catenin stimulator), lithium chloride (LiCl) and SB216763 (a GSK3β inhibitor) surprisingly enhance myelin gene expression *in vitro* (in cell lineages) and *in vivo* (in animals). These findings have opened new perspectives in the treatment of demyelinating diseases by administrating Wnt activators.

Wnt polypeptides are secreted cysteine-rich glycosylated polypeptides that play a role in the development of a wide range of organisms (Chien AJ et al, Journal of Investig. Dermatology 2009). The polypeptides of the Wnt family bind to an extracellular domain of a family of cell surface proteins called Frizzled receptors, and may play a role in embryonic induction, generation of cell polarity, and specification of cell fate. Wnts are encoded by a large gene family, whose members have been found in round worms, insects, cartilaginous fish and vertebrates. Wnts are thought to function in a variety of developmental and physiological processes since many diverse species have multiple conserved Wnt genes. The Wnt growth factor family includes at least 18 genes identified in the human by cDNA cloning (see in Miller JR, *Genome Biol* 2002). Wnts may play a role in local cell signaling and neurogenesis. Studies of mutations in Wnt genes have indicated a role for Wnts in growth control and tissue patterning. In Drosophila, wingless (wg) encodes a Wnt gene and wg mutations alter the pattern of embryonic ectoderm, neurogenesis, and imaginal disc outgrowth (Klingensmith J and Nusse R, *Dev Biol* 1994).

At the time of the present invention, it was currently thought that the Wnt signalling pathway has inhibitory effects on oligodendrocyte precursor differentiation, and has therefore a negative impact on the myelinisation (Shimizu et al, *Developmental Biology* 2005; Kim et al, *Developmental Dynamic* 2008).

In particular, the team of Fancy et al (*Genes and development,* 2009) showed the effect of the Wnt - β-catenin pathway in experimental remyelinating lesions in genetically altered mice. Interestingly, this study highlighted the role of the activated Tcf4 / β-catenin complex in the inhibition of the differentiation of the oligodendrocytes precursors. The authors finally suggested using pharmacological inhibitors of the pathway "for therapeutically enhancing the remyelination by normalising the kinetics of myelin repair". Also, Feigenson K et al (Molecular Cell Neuroscience 2009) showed that the Wnt pathway negatively affects the maturation of the oligodendrocytes precursors into myelinating oligodendrocytes, and therefore delays the myelination.

Therefore, at the time of the filing of the present application, no document enables the man skilled in the art to understand that Wnt signalling activators could be used for treating demyelinating conditions, especially CMT or PMD.

### BRIEF DESCRIPTION OF THE DRAWINGS

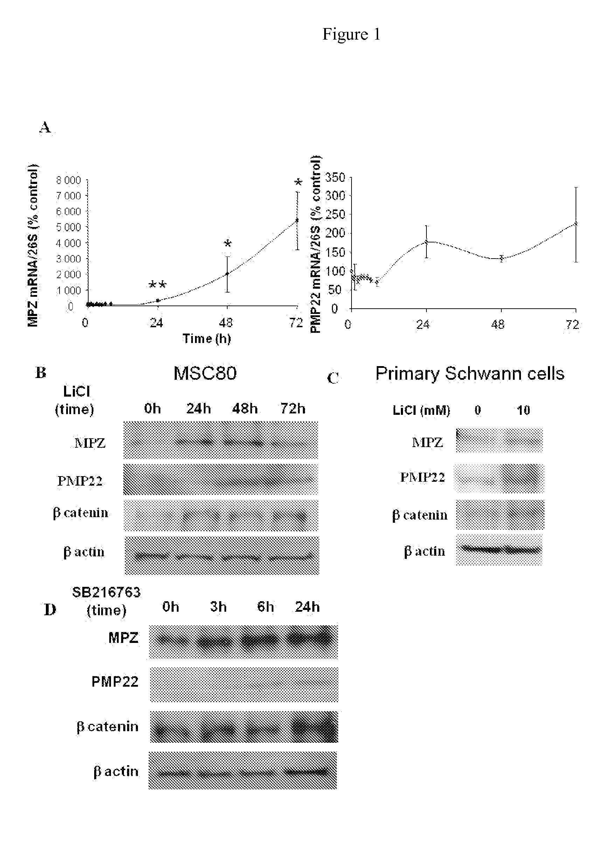

**Figure 1** shows the effect of LiCL (10 mM) on the expression of MPZ and PMP22 in MSC80 cells from 1 hour to 72 hours. (A) Quantitative real time PCR experiments performed on total RNA using primers recognizing MPZ or PMP22. *p<0.05, **p<0.01 by Mann-Whitney's test when compared to control. (B) Western Blots performed using either anti-MPZ, anti-PMP22 or anti-β-catenin antibodies on MSC80 cells. (C) Western Blots performed using either anti-MPZ, anti-PMP22 or antiβ-catenin antibodies on primary Schwann Cells treated with LiCL (10 or 100 mM) for 24 hours. (D) Western Blots performed using either anti-MPZ, anti-PMP22 or anti-β-catenin antibodies on MSC80 cells treated with SB216763 (10 µM) for 3, 6 or 24 hours.

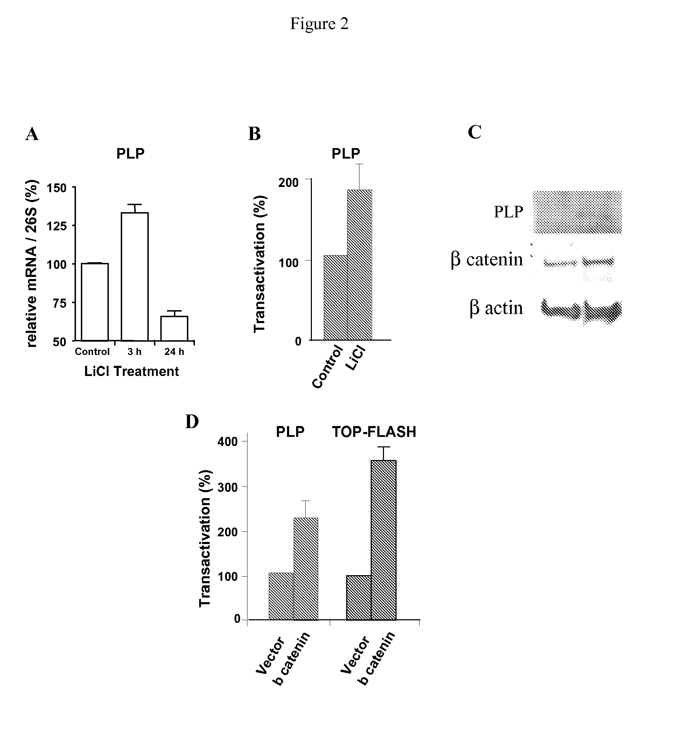

**Figure 2** shows the effect of LiCl (10mM) on the expression of PLP in 158N cells treated for 3 hours or 24 hours. (A) Quantitative real time PCR experiments were performed on total RNA using primers recognizing PLP. *p<0.05, **p<0.01 by Mann-Whitney's test when compared to control. (B) Analysis of β-galactosidase and luciferase activities in 158N cells transiently transfected with 0.2 µg of PLP-Luc, 0.1 µg of pRSV-β-Gal plasmids, and treated during 24h with LiCl (10 mM) 18 hours after transfection. (C) Western Blots performed on 158N cells using either anti-PLP or anti-β-catenin antibodies. (D) Analysis of β-galactosidase and luciferase activities of 158N cells 48 hours after transfection with 0.2 µg of PLP-Luc and 0.1 µg of pRSV-β-Gal plasmids, with or without β3-catenin expression vector.

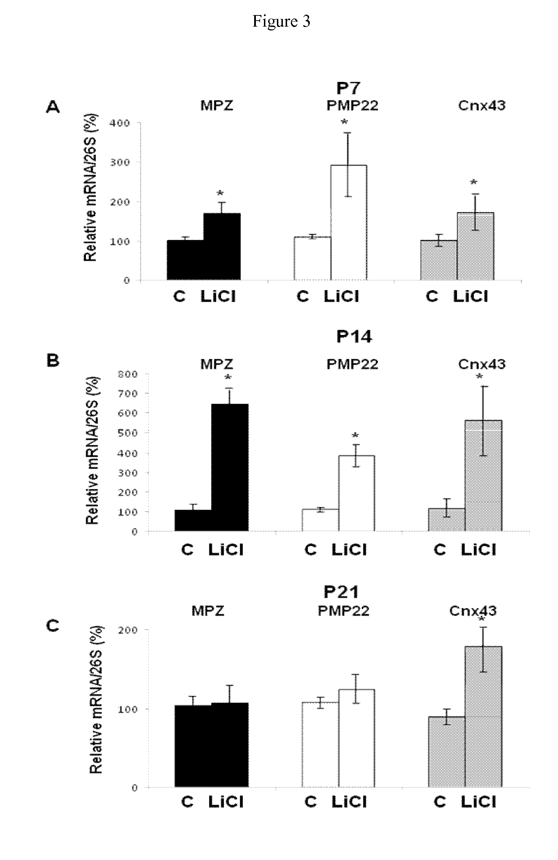

**Figure 3** shows the effect of LiCl on the expression of MPZ and PMP22 in developing sciatic nerves: quantitative real time PCR experiments were performed on total RNA using primers recognizing MPZ, PMP22 and Connexin 43 (Cnx43). *p<0.05 by Mann Whitney's test when compared to control.

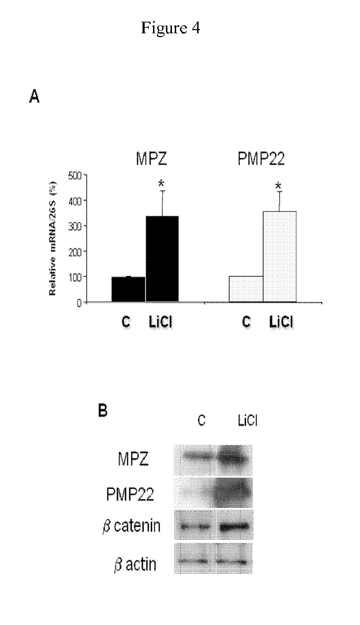

**Figure 4** shows the effect of LiCl on the expression of MPZ and PMP22 in adults mice. (A) Quantitative real time PCR experiments were performed on total RNA using primers recognizing MPZ or PMP22. *p<0.05 by Mann Whitney's test when compared to control. (B) Western Blots were performed using either anti-MPZ, anti-PMP22 or anti-βcatenin antibodies.

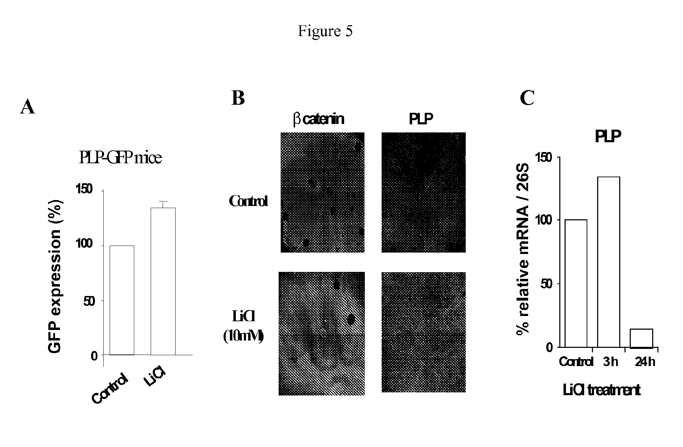

**Figure 5** shows the effect of LiCl on the expression of GFP in PLP-GFP adult mice. (A) The brains of 8-week old mice treated with LiCl (0.2%) per os during five days, were dissected and GFP fluorescence was analyzed and quantified using a fluorescent microscope. *p<0.05 by Mann Whitney's test when compared to control. (B) Cerebellar slices from P2 mice treated with LiCl (10 mM) during 24h and double-immunolabeled two days later with antibodies against PLP (red) and against β-catenin (green). (C) qRT-PCR experiments with primers recognizing specifically PLP, performed on cerebellar slices from P2 mice treated with LiCl (10 mM) during 3 or 24h.

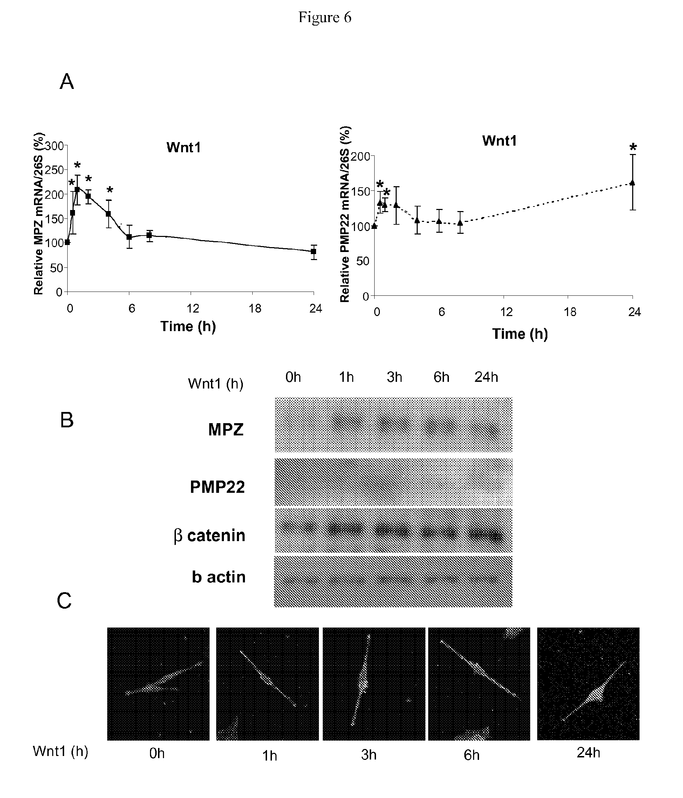

**Figure 6** shows the effect of Wnt1 on the expression of MPZ in MSC80 cells. (A) Quantitative real time PCR experiments performed using primers recognizing MPZ or PMP22 on total RNA extracted from MSC80 cells treated with Wnt1 (10 ng) from 1 to 24 hours. (B) Western Blots using either anti-MPZ, anti-PMP22, or anti-β-catenin antibodies (C) Immunocytochemistry experiments performed using an antibody directed against β-catenin on MSC80 cells incubated in the absence (0h) or in the presence of Wnt1 (10 ng) for 1 h, 3h, 6h or 24h.

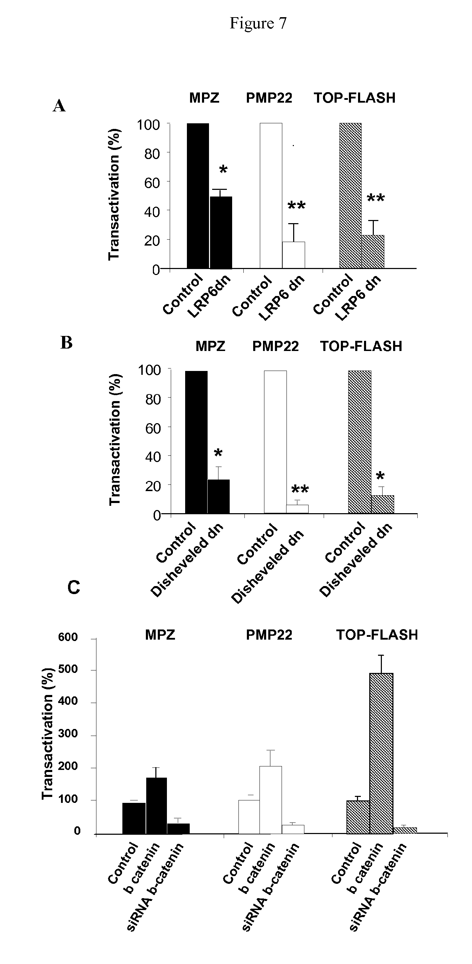

**Figure 7** shows the functional implication of Wnt signaling pathway in the basal expression of MPZ and PMP22. B-galactosidase and luciferase activities analysed in MSC80 cells 24 hours after transfection with MPZ-Luc, PMP22-Luc or TOP-FLASH-Luc plasmids and with one of the following plasmids: (A) LRP6dn, (B) Dshdn (C) either β-catenin expression vector or siRNA directed against β-catenin. *p<0.05, **p<0.01 by Mann Whitney's test when compared to control. (D) RT-PCR experiments performed on total RNA with primers recognizing specifically the four TCFs (TCF1, LEF1, TCF3 and TCF4). (E) Quantitative real time PCR experiments using primers recognizing specifically the four TCFs (TCF1, LEF1, TCF3 and TCF4) on total RNA from MSC80 cells transiently transfected with siRNA directed against the four TCFs or non targeting siRNA (NT). (F) β-galactosidase and luciferase activities analysed in MSC80 cells twenty-four hours after transfection with MPZ-Luc, PMP22-Luc or TOP-FLASH-Luc plasmids and with siRNA directed against the four TCFs or non targeting siRNA (NT) *p<0.05, **p<0.01 by Mann Whitney's test when compared to control.

### DETAILLED DESCRIPTION OF THE INVENTION

As shown by the experimental results presented hereafter, the present inventors have found that, contrarily to what was currently proposed in the literature (Fancy et al, Feigenson et al), the Wnt signalling pathway positively regulates the expression of myelin genes in Schwann cells and oligodendrocytes. More precisely, it was discovered that activators of the Wnt signalling pathway, for example Wnt 1, LiCl and SB216763, are able to enhance the expression of both peripheral and central myelin genes such as Myelin protein zero (MPZ), Peripheral Myelin Protein 22 KDa (PMP22), and Proteolipid Protein (PLP), either *in vitro* in cell lineages, and also *in vivo* in normal adult animals. This is particularly surprising, since the existing studies published so far had demonstrated that the activation of the Wnt-signalling pathway could delay the maturation of the oligodendrocyte precursors into myelinating cells.

Importantly, it has been established here for the first time that injecting Wnt pathway activators by a systemic route in newborn animals or locally in adult animals, can induce the expression of myelin genes in the peripheral (sciatic nerves) and central (cerebellum) nervous system in newborn and adult animals. This indicates that Wnt pathway activators can be used to enhance the developmental myelinisation (after birth) and to restore the adult myelinisation. These compounds can be used to counteract the deficient myelinisation associated with demyelinating disorders. Importantly, these compounds could enable to treat any myelin deficiency disorders, either of genetic cause (newborn treatment), or of viral and bacterial cause (adult treatment).

In a first aspect, the present invention is therefore drawn to the use of an effective amount of a Wnt, a Wnt-like substance, and/or a compound stimulating the Wnt signalling pathway,
i) for reducing the extent or the degree of nerve demyelination in the central and peripheric nervous system in an individual suffering from a demyelinating disorder,
ii) for increasing the myelination at or near a site of demyelination in the central and peripheric nervous system in an individual suffering from a demyelinating disorder,
iii) for treating a demyelinating disorder in an individual in need thereof,
iv) for inhibiting or reducing the symptoms of a demyelinating disorder in an individual in need thereof.

In other words, the present invention encompass methods for treating demyelinating disorders, for reducing the extent or the degree of nerve demyelination in the central and peripheric nervous system, for increasing the myelination at or near a site of demyelination in the central and peripheric nervous system, and/or for inhibiting or reducing the symptoms of a demyelinating disorder, comprising administering to an individual in need thereof an effective amount of a Wnt polypeptide, a Wnt-like substance, and/or a chemical compound stimulating the Wnt signalling pathway.

In the context of the invention, said individual can be a mammal, and is preferably a human.

A demyelinating disease is any disease of the nervous system in which the myelin sheath of neurons is damaged. This impairs the conduction of signals in the affected nerves, causing impairment in sensation, movement, cognition, or other functions depending on which nerves are involved. The term describes the effect of the disease or its cause; some demyelinating diseases are caused by genetics, some by infectious agents, some by autoimmune reactions, and some by unknown factors.

Myelin deficiency disorders and demyelinating disorders that can be treated with the subject method of the invention include Multiple Sclerosis (MS), optic neuritis, transverse neuritis, Guillain-Barre Syndrome (GBS), Pelizaeus-Merzbacher disease (PMD), Charcot-Marie-Tooth disease (CMT), leukodystrophies, extrapontine demyelination, acute disseminated encephalomyelitis, acute viral encephalitis, acute transverse myelitis, adrenoleukodystrophy, adrenomyeloneuropathy, AIDS-vacuolar myelopathy, HTLV-associated myelopathy, Leber's hereditary optic atrophy, progressive multifocal leukoencephalopathy, subacute sclerosing panencephalitis, and tropical spastic paraparesisintrapontine demyelination (also known as osmotic demyelination syndrome), and the like. Demyelination resulting from head injury or spinal cord trauma can also be treated with a subject method.

In a preferred embodiment, the present invention targets the demyelinating disorders called "leukodystrophies". In these diseases, the central symptom is the progressive disease of myelin: a genetically determined metabolic defect results in myelin alteration, which triggers the confluent destruction, or failed development, of central white matter (Berger J, *Current Opinion in Neurology* 2001). The leukodystrophies encompass the X-linked adrenoleukodystrophy (X-ALD), the Alexanders disease (AD), the Canavans disease (CD), the cerebrotendinous xanthomatosis (CTX), the globoid cell leukodistrophy (GLD, Krabbes disease), the metachromatic leukodystrophy (MLD), the Pelizaeus-Merzbacher disease (PMD), the polycystic lipomembranous osteodysplasia with sclerosing leukodystrophy (PLOSL), the Sjögren-Larsson syndrome (SLS) and some orphan genetic leukodystrophies such as the adult-onset autosomal-dominant leukodystrophy (ADLD), the vacuoliting megalencephalic leukodistrophy (VL) and the vanishing white matter disease.

In another preferred embodiment, the present invention targets the demyelinating diseases of the peripheral nervous system, that is the Guillain-Barre syndrome, the anti-MAG peripheral neuropathy, and the Charcot-Marie-Tooth disease (CMT).

The methods described above generally involve administering to an individual in need thereof an effective amount of a composition comprising Wnt polypeptide, a Wnt-like substance, and/or a chemical compound stimulating the Wnt signalling pathway. In some embodiments, an "effective amount" of a subject composition comprising a Wnt polypeptide, a Wnt-like substance, and/or a chemical compound stimulating the Wnt signalling pathway is an amount that is effective to enhance the myelin genes expression in the central or peripheric nervous system by at least about 10%, at least about 20%, a at least about 30%, at least about 40%, at least about 50%, at least about 60%, at least about 70%, at least about 80%, or at least about 90%, or more, compared to the level of gene expression in an untreated subject.

Symptoms of demyelinating disorders are known in the art. For example, symptoms of MS include difficulty walking; numbness ("pins and needles") in the extremities; pain on moving the eyes; dim or blurred vision, double vision, or loss of central vision; tremors (shakiness) ; clumsiness; slurred speech; poor memory; reduced cognitive abilities; painful muscle spasms; etc.

Any Wnt polypeptide is contemplated by the present invention. For example, the Wnt polypeptide may be a Wnt1 (e.g. the *homo sapiens* Wnt1, Accession Number: NP_005421, SEQ ID NO:11) , a Wnt2 (e.g. the *homo sapiens* Wnt2, Accession Number: NP_003382, SEQ ID NO:12), a Wnt2b (e.g. the *homo sapiens* Wnt2b, Accession Number: NP_004176, SEQ ID NO:13 or NP_078613, SEQ ID NO:14), a Wnt3 (e.g. the *homo sapiens* Wnt3, Accession Number: NP_110380, SEQ ID NO:15), a Wnt3a (e.g. the *homo sapiens* Wnt3a, Accession Number: NP_149122, SEQ ID NO:16), a Wnt4 (e.g. the *homo sapiens* Wnt4, Accession Number: NP_110388, SEQ ID NO:17), a Wnt5a (e.g. the *homo sapiens* Wnt5a, Accession Number: NP_003383, SEQ ID NO:18), a Wnt5b (e.g. the *homo sapiens* Wnt5b, Accession Number: NP_110402, SEQ ID NO:19), a Wnt 6 (e.g. the *homo sapiens* Wnt6, Accession Number: NP_006513, SEQ ID NO:20), a Wnt7a (e.g. the *homo sapiens* Wnt7a, Accession Number: NP_004616, SEQ ID NO:21), a Wnt7b (e.g. the *homo sapiens* Wnt7b, Accession Number: NP_478679, SEQ ID NO:22), a Wnt8a (e.g. the *homo sapiens* Wnt8a, Accession Number: NP_490645, SEQ ID NO:23), a Wnt8b (e.g. the *homo sapiens* Wnt8b, Accession Number: NP_003384, SEQ ID NO:24), a Wnt9a (e.g. the *homo sapiens* Wnt9a, Accession Number: NP_003386, SEQ ID NO:25), a Wnt9b (e.g. the *homo sapiens* Wnt9b, Accession Number: NP_003387, SEQ ID NO:26), a Wnt10a (e.g. the *homo sapiens* Wnt10a, Accession Number: NP_079492, SEQ ID NO:27), a Wnt10b (e.g. the *homo sapiens* Wnt10b, Accession Number: NP_003385 SEQ ID NO:28), a Wnt11 (e.g. the *homo sapiens* Wnt11, Accession Number: NP_004617, SEQ ID NO:29), or a Wnt16 (e.g. the *homo sapiens* Wnt16, Accession Number: NP_057171, SEQ ID NO:30 or NP_476509, SEQ ID NO:31) polypeptide. One of skill in the art would be familiar with the range of Wnts available that are contemplated by the present invention. In certain embodiments, the Wnt is a Wnt1, Wnt4, Wnt5a, Wnt6, or Wnt7b polypeptide. In certain embodiments, the Wnt polypeptide will be a mammalian Wnt protein, for example a human or murine Wnt polypeptide, or a homolog thereof from another vertebrate species. Commercial existing Wnt polypeptide L-M(TK-) cells producing active Wnt can be obtained from the ATCC (CRL2647, CRL2814).

The therapeutic use of Wnt polypeptides has already been proposed to promote hair growth (US6924141), haematopoiesis (US7335643), neurogenesis (US20080213892), oocyte viability (US6485972), neurone guidance (WO04103394). However, it has never been proposed to use Wnt polypeptide, for example Wnt1, Wnt4, Wnt5a, Wnt6, or Wnt7b polypeptide, to promote the expression of myelin genes and therefore the myelination.

In further embodiments, the present invention discloses the use of a Wnt-like substance for treating demyelinating disorders. This Wnt-like substance may be for example a Wnt peptide. Any Wnt peptide is contemplated by the present invention. For example, the Wnt peptide may be a Wnt1, Wnt2, Wnt2b, Wnt3, Wnt3a, Wnt4, Wnt5a, Wnt5b, Wnt 6, Wnt7a, Wnt7b, Wnt8a, Wnt8b, Wnt9a, Wnt9b, Wnt10a, Wnt10b, Wnt11, or Wnt16 peptide such as those selected in the group consisting of: SEQ ID NO: 11 to SEQ ID NO:31. One of skill in the art would be familiar with the range of Wnt peptides available that are contemplated by the present invention. In certain embodiments, the Wnt peptide is a Wnt1, Wnt4, Wnt5a, Wnt6, or Wnt7b peptide (SEQ ID NO:1, 17, 18, 20 or 22). In certain embodiments, the Wnt protein will be a mammalian Wnt peptide, for example a human Wnt peptide, or a homolog thereof from another vertebrate species. In other embodiments, the Wnt-like substance is a mimetic of Wnt or a mutant Wnt. Any Wnt mimetic is contemplated by the present invention. For example, the Wnt mimetic may be a Wnt1, Wnt2, Wnt2b, Wnt3, Wnt3a, Wnt4, Wnt5a, Wnt5b, Wnt 6, Wnt7a, Wnt7b, Wnt8a, Wnt8b, Wnt9a, Wnt9b, Wnt10a, Wnt10b, Wnt11, or Wnt16 mimetic. One of skill in the art would be familiar with the range of Wnt mimetics available that are contemplated by the present invention. In certain embodiments, the Wnt mimetic is a Wnt1, Wnt4, Wnt5a, Wnt6, or Wnt7b mimetic. In certain embodiments, the Wnt mimetic will be a mammalian Wnt mimetic, for example a human Wnt mimetic, or a homolog thereof from another vertebrate species. Any Wnt mutant that is active as a Wnt is contemplated by the present invention. For example, the Wnt mutant may be a Wnt1, Wnt2, Wnt2b, Wnt3, Wnt3a, Wnt4, Wnt5a, Wnt5b, Wnt 6, Wnt7a, Wnt7b, Wnt8a, Wnt8b, Wnt9a, Wnt9b, Wnt10a, Wnt10b, Wnt11, or Wnt16 mutant. One of skill in the art would be familiar with the range of Wnt mutants available that are contemplated by the present invention. In certain embodiments, the Wnt mutant is a Wnt1, Wnt4, Wnt5a, Wnt6, or Wnt7b mutant. In certain embodiments, the Wnt mutant will be a mammalian Wnt mutant, for example a human Wnt mutant, or a homolog thereof from another vertebrate species. In other embodiments, the Wnt-like substance is a small molecule.

Further embodiments of the present invention involve use of chemical compounds stimulating the Wnt signaling pathway to enhance the expression of myelin genes. One of ordinary skill in the art would be familiar with the wide range of such compounds available which can stimulate the Wnt signaling pathway.

Different methods are known to activate the Wnt signaling pathway: the first method is to use direct activators of the Wnt pathway, and the second is to use indirect activators such as inhibitors of Wnt-pathway inhibitors.

As direct activators, there are proteins unrelated to Wnt, such as norrin (Xu et al, *Cell* 2004) and R-spondin2 (Kazanskaya et al, *Dev Cell* 2004 ; Kim et al, *Science* 2005) that can also activate the Wnt pathway. Norrin binds to Frizzled4 (but only to that member of the Frizzled family); while R-spondin2 interacts with Frizzled8 (Nam et al, *J. Biol Chem,* 2006) and LRP6 (Wei et al, *J. Biol Chem,* 2007).

One well-known inhibitor of the Wnt-pathway is the glycogen synthase kinase 3 (GSK3). Therefore, in one embodiment, the present invention is drawn to the use of an inhibitor of GSK3 for treating demyelinating disorders.

GSK3 inhibitors have already been proposed in pharmaceutical compositions intended to treat for example type 2 diabetes, protozoan diseases, neuronal diseases, and circadian rhythm disease (Meijer L, *Trends Pharmacol Sci.* 2004).

One of these inhibitors is Lithium, in particular its salt lithium chloride (LiCI), which is well-known to activate the Wnt signaling (Klein PS and Melton DA, *PNAS* 1996). Lithium is commonly used in the treatment of bipolar disease as a mood stabilizer (Gould TD, *Neuropsychopharmacology* 2004). Recent *in vitro* and *in vivo* studies have demonstrated that lithium can be used in the treatment of acute brain injuries (ischemia, brain trauma) and neurodegenerative diseases (Parkinson's disease, Alzheimer disease, Multiple Sclerosis and Huntington's disease) (Zhong J and Lee WH, *Expert Opin Drug* saf 2007). As a matter of fact, lithium has a neuroprotective effect by preventing neuronal death and stimulating neurogenesis (Chuang DM, *Crit Rev neurobiol* 2004). Lithium acts by means of several mechanisms, either the inhibition of excitotoxicity by blocking NMDA receptor or the induction of the survival pathway by blocking Glycogen Synthase Kinase 3 β (GSK3β) which is a key-enzyme intervening in Wnt/β-catenin, Akt (Klein and Melton, *PNAS* 1996), and protein kinases A (Mori et al., *Life Science* 1996) and C pathway (Einat et al., *Neuropsychobiology.* 2007).

It is important to note that, despite its well-known effect on neuronal growth, lithium has never been shown to enhance the expression of myelin genes.

At least 30 other inhibitors of GSK3 have been described (cf table 1). They are, for example: hymenialdisine, flavopiridol, kenpaullone, alsterpaullone, 1-azakenpaullone, indirubin-3'-oxime, 6-bromoindirubin-3'oxime (BIO), 6-bromoindirubin-3'acetoxime, aloisine A and B, TDZD8, pyrazolopyridine 18, 34 and 9, CHIR98014, CHIR99021 (CT99021), CT20026, SU9516, ARA014418, staurosporine, GF109203x, Ro318220, SB216763, SB415286, I5, CGP60474, TWS119, compound 5a (bisindolylmaleimide), compound 29 (azaindolylmaleimide), compound 46 (azaindolylmaleimide), compound 1A (pyrazolopyrimidine) and beryllium.

| **Molecule name** | **Other chemical name** | **CAS number** | **Formula** | **reference** | **Supplyer** |
|---|---|---|---|---|---|
| **Hymenialdisine** | | 92005-12-7 | C₁₁H₁₀BrN₅O | | |
| **Flavopiridol** | | 146426-40-6 | C₂₁H₂₀ClNO₅ | | Shanghai Yingxuan Chempharm Co, Ltd |
| **kenpaullone** | | 142273-20-9 | C₁₆H₁₁BrN₂O | | Tocris |
| **Alsterpaullone** | | 237430-03-4 | C₁₆H₁₁N₃O₃ | | 3B Pharmachem International |
| **1-Azakenpaullone** | 9-Bromo-7,12-dihydropyrido[3',2':2,3]azepino[4 ,5-b]indol-6(5H)-one | 676596-65-9 | C₁₅H₁₀BrN₃O | | Medical isotopes, fluorochem Ltd |
| **Indirubin-3'-oxime** | Indirubin-3'-monoxime ; 3-[1,3-Dihydro-3-(hydroxyimino)-2H-indol-2-ylidene]-1,3-dihydro-2H-indol-2-one | 160807-49-8 | C₁₆H₁₁N₃O₂ | | Tocris |
| **6-bromoindirubin-3'oxime** | B1686 BIO; (2'Z,3'E)-6-Bromoindirubin-3'-oxime | 667463-62-9 | L₁₆H₁₁BrN₃O₂ | | Sima Aldrich |
| **6-bromoindirubin-3'acetoxime** | BIO-Acetoxime (2'*Z*,3'*E*)-6-Bromoindirubin-3'-acetoxime | | | Knockaert, M., et al. 2004. Oncogene 23, 4400. Polychronopoulos, P., et al. 2004. J. Med. Chem. 47, 935. Meijer, L., et al. 2003. Chem. Biol. 10, 1255. | EMD biosciences |
| **Aloisine A** | 7-n-butyl-6-(4-hydroxyphenyl)-5H-pyrrolo [2,3-b]pyrazine | 496864-16-5 | | | Merck |
| **Aloisine B** | | | | Mettey, Y., Gompel, M., Thomas, V., Garnier, M., Leost, M., Ceballos-Picot, I., Noble, M., Endicott, J., Vierfond, J. M. and Meijer, L. (2003). Aloisines, a new family of CDK/GSK-3 inhibitors. SAR study, crystal structure in complex with CDK2, enzyme selectivity, and cellular effects. J. Med. Chem. 46, 222-236. | |
| **TDZD8** | -Benzyl-2-methyl-1,2,4-thiadiazolidine-3,5-dione | 327036-89-5 | C₁₀H₁₀N₂O₂S | | Sigma Aldrich |
| **Pyrazolopyridine 18** | | | | Witherington, J. et al. (2003) 5-Aryl-pyrazolo[3,4-b]pyridines: potent inhibitors of glycogen synthase kinase-3 (GSK-3). Bioorg. Med. Chem. Lett. 13, 1577-1580 | |
| **Pyrazolopyridine 9** | | | | Witherington, J. et al. (2003) 5-Aryi-pyrazolo[3,4-b]pyridazines: potent inhibitors of glycogen synthase kinase-3 (GSK-3). Bioorg. Med. Chem. Lett. 13, 1581-1584 | |
| **Pyrazolopyridine 34** | | | | Witherington, J. et al. (2003) 6-Aryl-pyrazolo[3,4-b]pyridines: potent inhibitors of glycogen synthase kinase-3 (GSK-3). Bioorg. Med. Chem. Lett. 13, 3055-3057 ; Witherington, J. et al. (2003) 6-Heteroaryl-pyrazolo[3,4-b]pyridines: potent and selective inhibitors of glycogen synthase kinase-3 (GSK-3). Bioorg. Med. Chem. Lett. 13, 3059-3062 | |
| **CHIR98014** | CT 98014 | 252935-94-7 | C₂₀H₁₇Cl₂N₉O₂ | | Axon Medchem |
| **CHIR99021** | CT99021 | 252917-06-9 | C₂₂H₁₈Cl₂N₈ | | Axon Medchem |
| **CT20026** | CHIR637 | | | | Chiron corporation |
| **SU9516** | (Z)-1,3-Dihydro-3-(1 H-imidazol-4-ylmethylene)-5-methoxy-2H-indol-2-one | 377090-84-1 | C₁₃H₁₁N₃O₂ | | Tocris |
| **ARA014418** | | | | Bhat, R. et al. (2003) Structural insights and biological effects of glycogen synthase kinase 3-specific inhibitor AR-A014418, J. Biol. Chem. 278, 45937-45945 | |
| **staurosporine** | | 62996-74-1 | | | Cayman chemical |
| **GF109203x** | bisindolylmaleimide I | | | Hers, I. et al. (1999) The protein kinase C inhibitors bisindolylmaleimide I (GF 109203X) and IX (Ro 31-8220) are potent inhibitors of glycogen kinase-3 activity. FEBS Lett. 460, 433-436 | |
| **Ro318220** | BisindolylmaleimideIX | 125314-64-9 | C₂₅H₂₃N₅O₂S | | Tocris |
| **SB216763** | | 280744-09-4 | C₁₉H₁₂Cl₂N₂O₂ | | Cayman Chemical |
| **SB415286** | | 264218-23-7 | C₁₆H₁₀ClN₃O₅ | | Cayman chemical |
| **I-5** | 2- (4-Amino-furazan-3-yl)-benzoimidazol-1-yl]-acetic acid | | | Bertrand, J.A. et al. (2003) Structural characterization of the GSK-3b active site using selective and non-selective ATP-mimetic inhibitors. J. Mol. Biol. 333, 393-407 WO2003066629 | |
| **CGP60474** | 3-{4-[2-(3-chlorophenylamino)-pyrimidin-4-yl]-pyridin-2-yl-amino}-propanol | | | Peter Stanetty et al, Synthesis of analogs of the phenylamino-pyrimidine type protein kinase C inhibitor CGP 60474 utilizing a Negishi cross-coupling strategy Tetrahedron, Volume 62, Issue 10, 6 March 2006, Pages 2380-2387 | |
| **TWS119** | | 601514-19-6 | C₁₈H₁₄N₄O₂ | | Cayman chemical |
| **Compound 5a** | bisindolylmaleimide | | | Han-Cheng Zhang et al, Macrocyclic bisindolylmaleimides as inhibitors of protein kinase C and glycogen synthase kinase-3, Bioorganic & Medicinal Chemistry Letters Volume 13, Issue 18, 15 September 2003 Pages 3049-3053 | |
| **Compound 46** | azaindolylmaleimide | | | SHEN Lan et al, Synthesis and biological evaluation of novel macrocyclic bis-7-azaindolylmaleimides as potent and highly selective glycogen synthase kinase-3β (GSK-3β) inhibitors, Bioorganic & medicinal chemistry, 2004, vol. 12, n°5, pp. 1239-1255 | |
| **Compound 1A** | pyrazolopyrimidine | | | Meijer L et al, Pharmacological inhibitors of glycogen synthase kinase 3, TRENDS in Pharmacological Sciences Vol.25 No.9 September 2004 | |
| **Compound 29** | azaindolylmaleimide | | | Meijer L et al, Pharmacological inhibitors of glycogen synthase kinase 3, TRENDS in Pharmacological Sciences Vol.25 No.9 September 2004 | |
| **beryllium** | | 7440-41-7 | | | GFS chemical inc |

The more efficient GSK3 inhibitors are: 6-bromoindirubin-3'oxime (BIO), 6-bromoindirubin-3'acetoxime, pyrazolopyridine 9, 18 and 34, CHIR98014, CHIR99021, staurosporine, compound 5a (bisindolylmaleimide), compound 29 (azaindolylmaleimide), compound 46 (azaindolylmaleimide), Ro318220, SB216763, SB415286, CGP60474, TWS119, and compound 1A (pyrazolopyrimidine) (IC₅₀ below 0,1, see Meijer L et al, *Trends in Pharmacological Sciences* 2004).

The commercially available GSK3 inhibitors are: flavopiridol, kenpaullone, alsterpaullone, 1-azakenpaullone, indirubin-3'-oxime, 6-bromoindirubin-3'oxime (BIO), 6-bromoindirubin-3'acetoxime, aloisine A, TDZD8, CHIR98014, CHIR99021, CT20026, SU9516, staurosporine, Ro318220, SB216763, SB415286, TWS119, and beryllium (see example of suppliers in table 1).

Other GSK3 inhibitors can be blocking anti-GSK3 antibodies or a polynucleotide molecule comprising a sequence that is antisense to a nucleic acid encoding a GSK3, for example a small interfering RNA (siRNA) based on a nucleic acid encoding either GSK-3α or GSK-3β commercially available in Millipore, or Cell Signaling Technology for example).

Also, suitable GSK3 inhibitors useful in the present invention are those disclosed in WO09/017454, WO06/034207, WO03/066629, WO03/004472, WO03/055492, WO03/082853, WO06/001754, WO07/040438, WO 07/040439, and WO07/040440, WO08/002244 and WO08/02245 which are incorporated by reference.

In a preferred embodiment, the pharmaceutical compositions of the present invention comprises the GSK3 inhibitors selected among: 6-bromoindirubin-3'oxime (BIO), 6-bromoindirubin-3'acetoxime, CHIR98014, CHIR99021, staurosporine, Ro318220, SB216763, SB415286, and TWS119 that are currently commercially available and very efficient GSK3 inhibitory activity.

Blocking negative regulators of Wnt signaling, such as Axin and APC will also activate the pathway, and using RNAi is an obvious choice. Therefore, in one embodiment, the present invention is drawn to the use of an inhibitor of Axin or APC, preferably siRNA anti-Axin and siRNA anti-APC, for treating demyelinating disorders.

The Wnt, the Wnt-like substance, and/or the chemical compound stimulating the Wnt signaling pathway may include a fused amino acid sequence that is designed to facilitate incorporation of the polypeptide into the intracellular compartment of a cell. For example, the Wnt-like substance may include a polypeptide encoding an amino acid TAT sequence from HIV. In another example, the Wnt-like substance may include a polypeptide encoding an Antp amino acid sequence. In another example, the Wnt-like substance may include a polypeptide encoding a VP22 amino acid sequence from HSV.

In certain embodiments, the Wnt, the Wnt-like substance, and/or the chemical compound stimulating the Wnt signaling pathway further includes an expression cassette comprising a promoter, active in a cell, operably linked to a polynucleotide encoding the Wnt, Wnt- like substance, and/or chemical compound stimulating a Wnt signaling pathway. For example, the polypeptide may be a Wnt1, Wnt2, Wnt2b, Wnt3, Wnt3a, Wnt4, Wnt5a, Wnt5b, Wnt 6, Wnt7a, Wnt7b, Wnt8a, Wnt8b, Wnt9a, Wnt9b, Wnt10a, Wnt10b, Wnt11, or Wnt16 polypeptide. In certain embodiments, the Wnt polypeptide is a Wnt1, Wnt4, Wnt5a, Wnt6, or Wnt7b polypeptide. In other embodiments, the expression cassette is carried in a viral vector. Although any viral vector is contemplated by the present invention, examples include an adenoviral vector, a retroviral vector, an adeno-associated viral vector, a vaccinia viral vector, or a pox viral vector. In other embodiments, the expression cassette is carried in a nonviral vector, such as a liposome. One of skill in the art would be familiar with a wide range of viral and nonviral vectors available to be of use in the present invention. Any promoter is contemplated for use in the present invention, as long as it facilitates expression of the polynucleotide. One of skill in the art would be familiar with the wide range of promoters available. For example, the promoter may be a constitutive promoter, an inducible promoter, or a tissuespecific promoter.

Certain embodiments of the present invention involve obtaining the Wnt, Wnt-like substance, and/or chemical compound affecting a Wnt signaling pathway from media of cultured cells. Although any cultured cells are contemplated by the present invention, in certain embodiments the cultured cells comprise an expression cassette including a promoter, active in the cultured cells, operably linked to a polynucleotide encoding Wnt, a Wnt-like substance, and/or a chemical compound stimulating the Wnt signaling pathway. The characteristics of expression cassettes that have been previously discussed above apply to these embodiments of the present invention.

As used herein, the terms "treatment", "treating" and the like, refer to obtaining a desired pharmacologic and/or physiologic effect. The effect may be prophylactic in terms of completely or partially preventing a disease or symptom thereof and/or may be therapeutic in terms of a partial or complete cure for a disease and/or adverse affect attributable to the disease. "Treatment" as used herein, covers any treatment of a disease in a mammal, particularly in a human, and includes: (a) increasing survival time; (b) decreasing the risk of death due to the disease; (c) preventing the disease from occurring in a subject which may be predisposed to the disease but has not yet been diagnosed as having it; (d) inhibiting the disease, i. e., arresting its development (e. g., reducing the rate of disease progression) ; and (e) relieving the disease, i. e., causing regression of the disease. The term "treatment" also includes improving the quality of life.

The terms "individual", "host", "subject", and "patient" used interchangeably herein, refer to a mammal, including primates, rodents (e. g., mice, rats), livestock, mammalian pets, horses, etc. In some embodiments, an individual is a human.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications mentioned herein are incorporated herein by reference to disclose and describe the methods and/or materials in connection with which the publications are cited.

The publications discussed herein are provided solely for their disclosure prior to the filing date of the present application. Nothing herein is to be construed as an admission that the present invention is not entitled to antedate such publication by virtue of prior invention.

In one embodiment, the present invention targets the use of a pharmaceutical composition comprising the Wnt, the Wnt-like substance, and/or the chemical compound stimulating the Wnt signaling pathway for treating demyelinating disorders.

A wide variety of pharmaceutically acceptable excipients is known in the art and need not be discussed in detail herein. Pharmaceutically acceptable excipients have been amply described in a variety of publications, including, for example, A. Gennaro (2000) "Remington: The Science and Practice of Pharmacy", 20th edition, Lippincott, Williams, & Wilkins; Pharmaceutical Dosage Forms and Drug Delivery Systems (1999) H. C. Ansel et al. , eds 7th ed., Lippincott, Williams, & Wilkins; and Handbook of Pharmaceutical Excipients (2000) A.H. Kibbe et al., eds., 3rd ed. Amer. Pharmaceutical Assoc.

Suitable pharmaceutically acceptable carriers include, but are not limited to, water, salt solutions (e.g., NaCl), alcohols, gum arabic, vegetable oils, benzyl alcohols, polyethylene glycols, gelatin, carbohydrates such as lactose, amylose or starch, magnesium stearate, talc, silicic acid, viscous paraffin, perfume oil, fatty acid esters, hydroxymethylcellulose, and polyvinyl pyrolidone. The pharmaceutical preparations can be sterilized and if desired, mixed with auxiliary agents, e. g., lubricants, preservatives, stabilizers, wetting agents, emulsifiers, salts for influencing osmotic pressure, buffers, coloring, flavoring and/or aromatic substances and the like which do not deleteriously react with the active compounds. The composition, if desired, can also contain minor amounts of wetting or emulsifying agents, or pH buffering agents. The composition can be a liquid solution, suspension, emulsion, tablet, pill, capsule, sustained release formulation, or powder. The composition can be formulated as a suppository, with traditional binders and carriers such as triglycerides. Oral formulation can include standard carriers such as pharmaceutical grades of mannitol, lactose, starch, magnesium stearate, polyvinyl pyrollidone, sodium saccharine, cellulose, magnesium carbonate, etc.

The composition can be formulated in accordance with the routine procedures as a pharmaceutical composition adapted for intravenous administration to an individual. Typically, compositions for intravenous administration are solutions in sterile isotonic aqueous buffer.

Where necessary, the composition may also include a solubilizing agent and a local anesthetic to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water, saline or dextrose/water. Where the composition is administered by injection, an ampoule of sterile water for injection or saline can be provided so that the ingredients may be mixed prior to administration.

A subject composition comprises, as active agents, the Wnt, the Wnt-like substance, and/or the chemical compound stimulating a Wnt signaling pathway. These active reagents are in some embodiments formulated into preparations for injection by dissolving, suspending or emulsifying them in an aqueous or nonaqueous solvent, such as vegetable or other similar oils, synthetic aliphatic acid glycerides, esters of higher aliphatic acids or propylene glycol; and if desired, with conventional additives such as solubilizers, isotonic agents, suspending agents, emulsifying agents, stabilizers and preservatives. The active agents can be formulated as neutral or salt forms. Pharmaceutically acceptable salts include those formed with free amino groups such as those derived from hydrochloric, phosphoric, acetic, oxalic, tartaric acids, etc. , and those formed with free carboxyl groups such as those derived from sodium, potassium, ammonium, calcium, ferric hydroxides, isopropylamine, triethylamine, 2-ethylamino ethanol, histidine, procaine, etc.

For oral preparations, the active agents (e.g., the Wnt, the Wnt-like substance, and/or the chemical compound stimulating a Wnt signaling pathway) are formulated alone or in combination with appropriate additives to make tablets, powders, granules or capsules, for example, with conventional additives, such as lactose, mannitol, corn starch or potato starch; with binders, such as crystalline cellulose, cellulose derivatives, acacia, corn starch or gelatins; with disintegrators, such as corn starch, potato starch or sodium carboxymethylcellulose; with lubricants, such as talc or magnesium stearate; and if desired, with diluents, buffering agents, moistening agents, preservatives, and flavoring agents.

Furthermore, the active agents can be made into suppositories by mixing with a variety of bases such as emulsifying bases or water-soluble bases. The suppository can include vehicles such as cocoa butter, carbowaxes and polyethylene glycols, which melt at body temperature, yet are solidified at room temperature. Unit dosage forms for oral or rectal administration such as syrups, elixirs, and suspensions may be provided wherein each dosage unit, for example, teaspoonful, tablespoonful, tablet or suppository, contains a predetermined amount of the composition containing one or more active agents. Similarly, unit dosage forms for injection or intravenous administration may comprise the active agents in a composition as a solution in sterile water, normal saline or another pharmaceutically acceptable carrier.

In another embodiment, the pharmaceutical composition of the present invention further comprises at least one additional therapeutic agent for treating a demyelinating disorder, for example an interferon-β, or a corticosteroid, preferably selected from methylprednisolone, prednisone, prednisolone, and dexamethasone.

### EXAMPLES

### Example 1: Expression of the components of the Wnt/β-catenin signaling pathway in sciatic nerve, in mouse Schwann cells and in oligodendrocytes.

To analyze the effect of Wnt/β-catenin signaling pathway on the expression of myelin genes, the mouse Schwann cell line (MSC80) and mouse oligodendrocytes line (158N) have been used. As a matter of fact, MSC80, to the difference of primary Schwann cells, and 158N cells are grown in a standard culture medium (DMEM) containing fetal calf serum and devoid of any steroid hormone (dexamethasone, progesterone), PKA activators (Forskolin) or added growth factors. This allows the evaluation of the effect of Wnt signaling pathway on myelin gene expression without any interference or cross-talk with other signaling pathways. These cells were analyzed by immunohistochemistry.

Briefly, cells were fixed in 4% paraformaldehyde in phosphate buffer (0.1 M, pH 7.4) for 1 h at room temperature. After washing in phosphate-buffered saline (PBS), slices were taken off the Millicell membranes and processed for immunocytochemistry. In all cases, slices were incubated for 1 h in phosphate-buffered saline 0.12M (pH 7.4) containing 0.25% Triton X-100, 0.2% gelatin, 0.1% sodium azide (PBSGTA) and lysine (0.1 M), before the overnight incubation with the primary antibodies (diluted in PBSGTA: anti-PLP 1/100 and anti-β-catenin 1/500). The following secondary antibodies were used: goat antirabbit Cy3 (1/250 dilution; Jackson ImmunoResearch Laboratories, Inc., West Baltimore Pike, USA), and goat anti-mouse Alexa Fluor488 (1/500 dilution, Molecular Probes, Leiden, Netherlands). After 2 h incubation, slices were washed several times in PBS, mounted with mowiol (Calbiochem) and analyzed using a fluorescent microscope (Axiovert 135M Zeiss, Carl Zeiss Inc., Germany).

Wnt1 and LRP6 showed a membrane and cytoplasmic staining. Dsh and GSK3β were cytoplasmic. β-catenin elicited a membrane and cytoplasmic staining but a small fraction was nuclear. As expected, the transcription factors TCF3/4 and LEF1 were strictly nuclear.

These data showed that the major components of Wnt/β-catenin signaling pathway were all expressed in mouse Schwann cells and in oligodendrocytes.

### Example 2: Wnt1 stimulates the expression of myelin gene expression in Schwann cells

MSC80 cells were incubated with recombinant Wnt1 ligand (10ng) from 1 to 24h (Wnt1 , Clinisciences, France) and quantitative real time PCR performed:
Total RNA from cultured MSC80 and from Primary Schwann Cells was obtained using RNA NOW (Ozyme, France). One µg was reverse transcribed with random primers from Promega (Charbonnières, France) and reverse transcriptase M-MLV-RT from Invitrogen (Cergy Pontoise, France). PCR experiments were performed using Taq DNA polymerase purchased from Promega (Charbonnières, France) and primers specific to each gene from Operon (Cologne, Germany). PCR products were analyzed on agarose gel (2%) and visualized under UV.

Quantitative real time PCR was performed with standard protocols using SYBR®Green ROX Mix (ABgene, France) as a fluorescent detection dye in ABI PRISM® 7000 in a final volume of 10 µl which also contained 300 nM primers (Operon, Cologne, Germany) and 20 ng of reverse transcribed RNA in 384-well plates. To characterize the generated amplicons and to control the contamination by unspecific by-products, a melting curve analysis was applied. Each reaction was performed in triplicate and the mean of at least three independent experiments was calculated. All results were normalized to the 26S mRNA level and calculated using the Delta Ct method. The primer sequences used in real time PCR are listed below:
MPZ Mouse F 5'GTCAAGTCCCCCAGTAGAA3' (SEQ ID NO1)
MPZ Mouse R 5'AGGAGCAAGAGGAAAGCAC3' (SEQ ID NO2)
MPZ Rat F 5'GTCCAGTGAATGGGTCTCAGA3' (SEQ ID NO3)
MPZ Rat R 5'CTTGGCATAGTGGAAGATTGAAA3' (SEQ ID NO4)
PMP22 Mouse and Rat F 5'AATGGACACACGACTGATC3'(SEQ ID NO5)
PMP22 Mouse and Rat R 5'CCTTTGGTGAGAGTGAAGAG3' (SEQ ID NO6)
PLP Mouse F : 5' AACTGCCTCTTTCTTCTTCC3' (SEQ ID NO 7)
PLP Mouse R : 5' ATGTCCTAGCCATTTTCCC3' (SEQ ID NO 8)
26S F 5'AGGAGAAACAACGGTCGTGCCAAAA3' (SEQ ID NO 9)
26S R 5'GCGCAAGCAGGTCTGAATCGTG3' (SEQ ID NO10)

Real-time PCR experiments showed that MPZ expression was enhanced 2-fold after 1 h of treatment, fell down after 3h of treatment (1.75-fold stimulation) and was finally abolished after 5h of treatment (Fig 6A). PMP22 expression was also stimulated, but to a lesser extent. Actually, Wnt1 treatment provoked a transient 40% increase in PMP22 transcripts after 30min and 1 h (Fig 6A).

Western blot analysis confirmed the stimulation of MPZ and PMP22 expression. As shown in Figure 6B, Wnt1 induced the expression of MPZ, PMP22 and β-catenin proteins after 1h of treatment, but contrary to mRNA, this stimulation lasted up to 24h of treatment. This difference may be due to specific mRNA and protein half-life.

Also, the effect of Wnt1 on the localization of β-catenin in MSC80 was studied by immunofluorescence (see experimental settings in example 1).

As depicted in Figure 6C, Wnt1 provoked a modification in MSC80 shape. In fact, after 1 hour of Wnt1 treatment, a prolongation of the MSC80 extensions is observed. Wnt1 also elicited a relocalization of β-catenin. After 1 to 6 hours of Wnt1 treatment, a fraction of β-catenin was localized in the plasma membrane and the other fraction was nucleocytoplasmic. After 24h of incubation with Wnt1, a clear relocalization of β-catenin in the nucleus is observed, whereas, in control cells, the major part of β-catenin was localized in the cytoplasm and only a little fraction was nuclear.

Interestingly, it is shown here for the first time that Wnt1 stimulates the expression of myelin gene expression in Schwann cells.

### Example 3: Each component of the Wnt / β-catenin pathway is required for the basal expression of myelin genes

Then the role of the canonical Wnt/β-catenin signaling pathway in the basal expression of myelin genes was investigated. To address this question, the expression of its major components including LRP6, Dsh, β-catenin and the TCF/LEF was altered by means of loss-of-function strategy.

First, the co-receptor LRP6 was blocked by a dominant negative form (LRP6 dn) in MSC80 cells (Fig. 7A). As shown in Figure 7A, LRP6dn inhibited by 80% the basal activity of TOP-FLASH, indicating that the dominant-negative mutant of LRP6 is effective. Subsequently, the MSC80 cells were cotransfected with MPZ-Luc or PMP22-Luc plasmids and LRP6dn expression vector. LRP6dn elicited an inhibition of 50% and 80% of the expression of MPZ and PMP22, respectively (Fig 7A), highlighting the crucial role of LRP6 co-receptor in the regulation of MPZ and PMP22 expression.

Then, a dominant-negative form of Dsh (Dshdn) was overexpressed in MSC80 cells. Dshdn was able to inhibit up to 90% the transactivation of TOP-FLASH promoter in the Schwann cell line, which confirms the efficiency of Dshdn (Fig 7B). Transfected with MPZ-Luc or PMP22-Luc constructs, Dshdn inhibited up to 80% and 90% MPZ and PMP22 promoter activities, respectively (Fig 7B). These results clearly demonstrate the pivotal role of Dsh in the regulation of peripheral myelin genes.

Two strategies were adopted to evaluate the implication of β-catenin in myelin gene expression: (i) overexpression of the nuclear form of β-catenin, (ii) knock-down of β-catenin expression by a specific siRNA.

Overexpression of the active nuclear form of β-catenin in MSC80 cells, first tested in the control TOP-FLASH-Luc construct, caused a 2-fold increase in the MPZ and PMP22 promoter activities (Fig 7C). The effect of β-catenin inhibition was next evaluated by using a specific siRNA, whose effectiveness was verified by immunofluorescent staining of β-catenin in MSC80 cells transfected or not with the siRNA (not shown). In the case of MPZ or PMP22 promoters, the knock-down of β-catenin expression provoked an 80%-inhibition of the transactivation (Fig 7C). As a control, the effect of the knock-down of the expression of β-catenin on the expression of the TOP-FLASH promoter was tested. The siRNA targeting β-catenin drastically inhibited the transactivation of the TOP-FLASH promoter (90%-inhibition) (Fig 7C), indicating that the designed siRNA is efficient.

Finally, the role of the final "relays" of the canonical Wnt signaling pathway, which are TCF/LEF transcription factors, were evaluated. First, it was shown that the four TCFs (TCF1, LEF1, TCF3 and TCF4) are all expressed in MSC80 cells (Fig 7D). Then, the expression of each TCF was knocked-down by using a selective siRNA. The siRNAs directed against the four TCFs (TCF1, LEF1, TCF3 and TCF4) and non targeting siRNA (NT) were purchased from Dharmacon (Lafayette, CO). Each TCF was targeted with four different sequences of siRNA directed against four different regions of the cognate mRNA. The siRNA efficacy was assessed by real time PCR. Each siRNA inhibited the corresponding TCF (Fig. 7E). The effectiveness of these siRNA were further verified on the transactivation of the TOP-FLASH promoter. As shown in Fig. 7F, the four siRNAs directed against LEF/TCFs dampened the activity of the TOP-FLASH promoter (70% inhibition).

The knock-down of TCF3 and TCF4 inhibited by 75% and 80% the transactivation of MPZ promoter, respectively, while TCF1 and LEF1 exerted modest inhibitory effects (50% inhibition) (Fig 7F). In the case of PMP22, the knock-down of the four TCFs had equivalent effects (50% decrease in the basal expression) (Fig 7F).

Taken together, these data show that the major components of the Wnt signaling pathway (LRP6, Dsh, GSK3β, β-catenin and the TCFs) are required for the basal expression of the peripheral myelin genes, confirming the impact of the Wnt/β-catenin pathway on those genes.

### Example 4: Pharmacological inhibitors of GSK3β (LiCl and SB216763) activate myelin genes

Two different strategies to inhibit GSK3β have been adopted; either with LiCl (IC₅₀=10 mM), or with the more potent and selective inhibitor SB216763 (IC₅₀=34 nM). MSC80 cells have been treated with LiCl (10mM). There was no significant effect of this drug on MPZ or PMP22 mRNA from 1h until 8h of treatment, but LiCl stimulated by 3-fold MPZ transcript after 24 hours of incubation (Fig 1A). Interestingly, LiCl stimulation lasted longer. As a matter of fact, when MSC80 cells were incubated with LiCl for 48h and 72h, a stimulation of MPZ transcript respectively by 20-fold and 54-fold was observed (Fig. 1A). Stimulation of PMP22 transcript (2.5-fold) was also observed but to a lesser extent than MPZ (Fig. 1A).

The stimulatory effect of LiCl on MPZ and PMP22 proteins was confirmed by Western blot. As shown in figure 1B, after a 24h and 48h of LiCl treatment, MPZ protein levels were enhanced, then this stimulation fell-down after 72h of treatment. The increase in PMP22 protein expression was timedependent. LiCl also stimulated the expression of β-catenin which indicates the efficiency of the drug in inhibiting GSK3β. Similarly, LiCl was able to enhance myelin gene expression in Schwann cell primary cultures (figure 1 C). As depicted in figure 1C, LiCl augmented protein expression of MPZ, PMP22 and β-catenin in Schwann cells to the same extent than the MSC80 cell line. The inhibition of GSK3β by SB216763 gave similar results. MSC80 cells were incubated with SB216763 during 3h, 6h or 24h. The expression of MPZ, PMP22 and β-catenin proteins was enhanced after 3h of SB216763 treatment and lasted until 24h (Fig 1 D).

As far as PLP gene is concerned, it was also demonstrated that LiCl treatment (10mM) of the oligodendrocytes cell line (158N) induced a transitory stimulation of PLP mRNA expression at 3h (1.3 fold) which was completely abolished at 24h (0.6 fold) (figure 2A). Protein analysis by Western blot confirmed that this transient stimulation of PLP mRNA was sufficient to increase PLP protein level (figure 2C). Moreover, LiCl effects are exerted directly on PLP promoter activity since the treatment of 158N cells transfected with PLP-Luc with LiCl (10mM) increased by 1.8 fold PLP promoter activity (figure 2B). Moreover, overexpression of β-catenin activates 2.5 fold PLP promoter activity (Figure 2D).

In conclusion, these results demonstrate activation of Wnt/β-catenin can enhance the expression of the myelin genes PMP22, MPZ and PLP in a Schwann cell line, a oligodendrocyte cell line and in Schwann primary cells.

### Example 5: LiCl stimulates myelin genes expression in vivo during myelination

In order to mime the canonical Wnt/β-catenin signaling pathway (Hedgepeth et al., 1997), LiCl was used. Newborn mice (P2) were treated by *ip* injections of LiCl (50mg/kg/day) during 5 days. Next, sciatic nerves were extracted from P7, P14 and P21 animals and examined the expression of MPZ and PMP22 transcripts by qPCR. Connexin 43 transcript was used as a control target gene for LiCl in nerve.

LiCl stimulated the expression of MPZ by 70% and PMP22 by 3-fold in P7 animals (Fig. 3A). Connexin 43 transcript was increased by 70% which indicates the efficacy of the treatment. At P14, the effects of LiCl administration were much more potent. As a matter of fact, LiCl enables a 6.5-fold stimulation of MPZ transcript, a 3.8-fold enhancement of PMP22 and a 5.6-fold increase of the control connexin 43 gene (Fig. 3B). Finally, at P21, the effects of LiCl fell down. It was not observed any significant effect of LiCl on MPZ or PMP22 expression, although Connexin 43 transcript was still enhanced by 80% at P21 (Fig 3C).

These results show here for the first time that LiCl stimulates myelin genes expression *in vivo* during developmental myelination.

### Example 6: LiCl stimulates myelin genes expression in adult animals

The effects of LiCl on adult mice were also tested. As it as previously been mentioned, myelin gene expression is decreased in adulthood to avoid hyper or hypo/myelination. Therefore, 8-weeks old mice were treated with LiCl (0.2%) *per os* during five days, then were dissected the sciatic nerves were dissected, and the total RNA were extracted to assay the expression of MPZ and PMP22 transcripts by real time PCR. As shown in figure 4A, LiCl treatment stimulates by 3-fold the MPZ and PMP22 mRNAs. Western blot analysis confirm these results, as shown in figure 4B: LiCl enables the increase of the expression of MPZ, PMP22 and β-catenin proteins in the sciatic nerves of adult mice.

Also, the effect of LiCl on the expression of PLP in the CNS of adult animals was assessed by examining transgenic mice expressing a GFP (green fluorescent protein) transgene under the control of the PLP promoter. For this purpose, 8-weeks old PLP-GFP mice were treated with LiCl (0.2%) *per os* during five days. Figure 5A shows that LiCl elicits a 35% augmentation of PLP promoter activity in PLP-GFP mice. This indicates that lithium can enhance PLP gene expression *in vivo* in adult animal.

Organotypic slices is a powerful *ex vivo* model for studying myelination. Therefore, cerebellar slices were prepared from P2 FVB mice (Janvier, Le Genest St Isle, France). For each experiment, at least three animals and 18 slices were used. All procedures concerning animal care and use were carried out in accordance with the European Community Council Directive (86/609/EEC) and were approved by the animal care and use committee at the institute. After decapitation, brains were dissected out into cold Gey's balanced salt solution containing 5 mg/ml glucose (GBSS-Glu) and meninges were removed. Cerebellar parasagittal slices (350µm thick) were cut on a Macllwain tissue chopper and transferred onto membranes of 30mm Millipore culture inserts with 0.4µm pore size (Millicell, Millipore, Bedford, MA, USA). Slices were maintained in culture in six-well plates containing 1 ml of medium at 35°C in an atmosphere of humidified 5% CO2. The medium was composed of 50% basal medium with Earle's salts (Invitrogen, Gaithersburg, MD, USA), 2.5% Hanks' balanced salts solution (Life Technologies), 25% horse serum (Life Technologies), L-glutamine (1 mM) and 5 mg/ml glucose. One day after culture, cerebellar slices were treated with LiCl (10mM) during 24h. For RNA experiments, slices were lysed after treatment. For protein experiment and IHC, slices were maintained two additional days in culture.

To examine whether LiCl can enhance PLP expression, organotypic slices of P2 mouse cerebellum were therefore treated with LiCl (10mM) during 24h, after which an immunostaining of β-catenin and PLP proteins are realized. Figure 5B clearly shows an increase of PLP staining accompanied with the increase of β-catenin staining in lithium treated cerebellar slices. Moreover, LiCl leads to a 30% stimulation of PLP mRNAs after 3h of treatment (figure 5C). This stimulation was transient, since after 24h of LiCl treatment, PLP mRNAs were inhibited up to 90%. Those results clearly show that lithium can induce PLP expression in cerebellar slices.

### BIBLIOGRAPHY

Berger J, Current Opinion in Neurology 2001 Jun;14(3):305-12. Review.
Chien AJ et al, Journal of Investig. Dermatology 2009 Jul; 129(7):1614-27.
Chuang DM, Crit Rev neurobiol 2004; 16(1-2):83-90. Review
Désarnaud F. et al., Brain research 2000, May 19;865(1):12-6.
Einat et al., Neuropsychobiology. 2007;55(3-4):123-31.
Fancy et al. Genes and development, 2009 Jul 1;23(13):1571-85.
Feigenson K et al Molecular Cell Neuroscience 2009
Gould TD, Neuropsychopharmacology 2004 Jan;29(1):32-8
Kaya F, Neuromuscul disorder 2007 Mar;17(3):248-53.
Kazanskaya 2004 ; Dev Cell. 2004 Oct;7(4):525-34
Kim et al, 2005: Science. 2005 Aug 19;309(5738):1256-9
Kim et al, Developmental Dynamic 2008 Nov;237(11):3324-31
Klein PS and Melton DA, PNAS 1996. Aug 6;93(16):8455-9
Klingensmith J and Nusse R, DevBiol 1994 Dec;166(2):396-414.
Krajewski KM et al, Brain 2000, Jul;123 ( Pt 7):1516-27
Magnaghi V et al, J. Mol. Neuroscience 2007; 31(2):149-57.
Meijer L, Trends Pharmacol Sci. 2004 Sep;25(9):471-80
Meyer zu Hörste G et al, Ann. neurol. 2007 Jan;61(1):61-72
Miller JR, Genome Biol 2002 ;3(1):REVIEWS3001
Mori et al., Life Sci. 1996;59(9):PL99-104
Nam JS et al, 2006 J Biol Chem. 2006 May 12;281(19):13247-57.
Shimizu et al, Developmental Biology 2005; Jun 15;282(2):397-410.
Verrier DA Glia. 2009 Sep;57(12):1265-79
Wei Q et al, 2007 J Biol Chem. 2007 May 25;282(21):15903-11.
Xu et al, Cell. 2004 Mar 19;116(6):883-95
Zhong J and Lee WH, Expert Opin Drug saf 2007 Jul;6(4):375-83. Review.

## Claims

1. Use of an effective amount of a Wnt, a Wnt-like substance, and/or a compound stimulating the Wnt signalling pathway for the preparation of a pharmaceutical composition intended to reduce the extent or degree of nerve demyelination in the central and peripheric nervous system of an individual suffering from a demyelinating disorder.

2. Use of an effective amount of a Wnt polypeptide, a Wnt-like substance, and/or a chemical compound stimulating the Wnt signalling pathway in a pharmaceutical composition intended to treat a demyelinating disorder in an individual, or to inhibit or reduce the symptoms of demyelinating disorders.

3. Use of an effective amount of a Wnt polypeptide, a Wnt-like substance, and/or a chemical compound stimulating the Wnt signalling pathway in a pharmaceutical composition intended to increase the myelination at or near a site of demyelination in the central and peripheral nervous system.

4. The use according to claims 1 to 3, wherein said demyelinating disorder is chosen among multiple sclerosis, acute disseminated encephalomyelitis, acute viral encephalitis, acute transverse myelitis, adrenoleukodystrophy, adrenomyeloneuropathy, AIDS-vacuolar myelopathy, HTLV-associated myelopathy, Leber's hereditary optic atrophy, progressive multifocal leukoencephalopathy, subacute sclerosing panencephalitis, and tropical spastic paraparesis, Pelizaeus-Merzbacher disease (PMP) and Charcot-Marie-Tooth disease (CMT).

5. The use according to claims 1 to 4, wherein said Wnt polypeptide is Wnt 1, Wnt4, Wnt5a, Wnt6, or Wnt7b protein.

6. The use according to claims 1 to 4, wherein said Wnt-like substance is a Wnt polypeptide, a Wnt peptide or a mimetic of Wnt or a mutant Wnt:

7. The use according to claims 1 to 4, wherein said compound stimulating the Wnt signalling pathway is a GSK3 inhibitor.

8. The use according to claim 7 wherein said GSK3 inhibitor is a chemical compound chosen among: LiCl, 6-bromoindirubin-3'oxime (BIO), 6-bromoindirubin-3'acetoxime, pyrazolopyridine 9, 18 and 34, CHIR98014, CHIR99021, staurosporine, compound 5a (bisindolylmaleimide), compound 29 (azaindolylmaleimide), compound 46 (azaindolylmaleimide), Ro318220, SB216763, SB415286, CGP60474, TWS119, and compound 1A (pyrazolopyrimidine), or a pharmaceutically acceptable salt or derivative thereof.

9. The use according to claims 7 and 8 wherein said GSK3 inhibitor is the chemical compound SB216763.

10. The use according to claim 8, wherein said GSK3 inhibitor, is an anti-GSK-3β blocking antibody, a polynucleotide molecule comprising a sequence that is antisense to a nucleic acid encoding either GSK-3α or GSK-3β, or a small interfering RNA (siRNA) based on a nucleic acid encoding either GSK-3α or GSK-3β.

11. The use according to claim 1 to 10, wherein the demyelinating disorder is a leukodystrophy chosen among: the X-linked adrenoleukodystrophy (X-ALD), the Alexanders disease (AD), the Canavans disease (CD), the cerebrotendinous xanthomatosis (CTX), the globoid cell leukodistrophy (GLD, Krabbes disease), the metachromatic leukodystrophy (MLD), the Pelizaeus-Merzbacher disease (PMD), the polycystic lipomembranous osteodysplasia with sclerosing leukodystrophy (PLOSL), the Sjögren-Larsson syndrome (SLS) and some orphan genetic leukodystrophies such as the adult-onset autosomal-dominant leukodystrophy (ADLD), the vacuoliting megalencephalic leukodistrophy (VL) and the vanishing white matter diseaseper.

12. The use according to claim 1 to 10, wherein the demyelinating disorder is a demyelinating disease of the peripheral nervous system chosen among the Guillain-Barre syndrome, the anti-MAG peripheral neuropathy, and the Charcot-Marie-Tooth disease (CMT).

13. The use according to claims 1 to 12, wherein the pharmaceutical composition further comprises at least one additional therapeutic agent for treating a demyelinating disorder.

14. The use according to claims 1 to 13, wherein said individual is a mammal, preferably a human.


SEQUENCE LISTING

<110>  Centre National de la Recherche Scientifique (CNRS)

<120>  USE OF WNT STIMULATORS FOR TREATING DEMYELINATING DISEASES

<130>  68148_D27725

<160>  31

<170>  PatentIn version 3.3

<210>  1
<211>  19
<212>  DNA
<213>  artificial

<220>
<223>  MPZ Mouse Forward primer

<400>  1
gtcaagtccc ccagtagaa                                                    19


<210>  2
<211>  19
<212>  DNA
<213>  artificial

<220>
<223>  MPZ Mouse Reverse primer

<400>  2
aggagcaaga ggaaagcac                                                    19


<210>  3
<211>  21
<212>  DNA
<213>  artificial

<220>
<223>  MPZ Rat Forward primer

<400>  3
gtccagtgaa tgggtctcag a                                                 21


<210>  4
<211>  23
<212>  DNA
<213>  artificial

<220>
<223>  MPZ Rat Reverse primer

<400>  4
cttggcatag tggaagattg aaa                                               23


<210>  5

<211>  19
<212>  DNA
<213>  artificial

<220>
<223>  PMP22 Mouse and Rat Forward primer

<400>  5
aatggacaca cgactgatc                                                    19


<210>  6
<211>  20
<212>  DNA
<213>  artificial

<220>
<223>  PMP22 Mouse and Rat Reverse primer

<400>  6
cctttggtga gagtgaagag                                                   20


<210>  7
<211>  20
<212>  DNA
<213>  artificial

<220>
<223>  PLP Mouse Forward primer

<400>  7
aactgcctct ttcttcttcc                                                   20


<210>  8
<211>  19
<212>  DNA
<213>  artificial

<220>
<223>  PLP Mouse Reverse primer

<400>  8
atgtcctagc cattttccc                                                    19


<210>  9
<211>  25
<212>  DNA
<213>  artificial

<220>
<223>  26S Forward primer

<400>  9
aggagaaaca acggtcgtgc caaaa                                             25


<210>  10
<211>  22

<212>  DNA
<213>  artificial

<220>
<223>  26S Reverse primer

<400>  10
gcgcaagcag gtctgaatcg tg                                                22


<210>  11
<211>  370
<212>  PRT
<213>  homo sapiens


<220>
<221>  misc_feature
<223>  Wnt 1 homo sapiens NP_005421

<400>  11

<210>  12
<211>  360
<212>  PRT
<213>  homo sapiens

<220>
<221>  misc_feature
<223>  Wnt2 homo sapiens NP_003382

<400>  12

Met Asn Ala Pro Leu Gly Gly Ile Trp Leu Trp Leu Pro Leu Leu Leu
1               5                   10                  15

Thr Trp Leu Thr Pro Glu Val Asn Ser Ser Trp Trp Tyr Met Arg Ala
            20                  25                  30

Thr Gly Gly Ser Ser Arg Val Met Cys Asp Asn Val Pro Gly Leu Val
        35                  40                  45

Ser Ser Gln Arg Gln Leu Cys His Arg His Pro Asp Val Met Arg Ala
    50                  55                  60

Ile Ser Gln Gly Val Ala Glu Trp Thr Ala Glu Cys Gln His Gln Phe
65                  70                  75                  80

Arg Gln His Arg Trp Asn Cys Asn Thr Leu Asp Arg Asp His Ser Leu
                85                  90                  95

Phe Gly Arg Val Leu Leu Arg Ser Ser Arg Glu Ser Ala Phe Val Tyr
            100                 105                 110

Ala Ile Ser Ser Ala Gly Val Val Phe Ala Ile Thr Arg Ala Cys Ser
        115                 120                 125

Gln Gly Glu Val Lys Ser Cys Ser Cys Asp Pro Lys Lys Met Gly Ser
    130                 135                 140

Ala Lys Asp Ser Lys Gly Ile Phe Asp Trp Gly Gly Cys Ser Asp Asn
145                 150                 155                 160

Ile Asp Tyr Gly Ile Lys Phe Ala Arg Ala Phe Val Asp Ala Lys Glu
                165                 170                 175

Arg Lys Gly Lys Asp Ala Arg Ala Leu Met Asn Leu His Asn Asn Arg
            180                 185                 190

Ala Gly Arg Lys Ala Val Lys Arg Phe Leu Lys Gln Glu Cys Lys Cys
        195                 200                 205

His Gly Val Ser Gly Ser Cys Thr Leu Arg Thr Cys Trp Leu Ala Met

210                 215                 220

Ala Asp Phe Arg Lys Thr Gly Asp Tyr Leu Trp Arg Lys Tyr Asn Gly
225                 230                 235                 240

Ala Ile Gln Val Val Met Asn Gln Asp Gly Thr Gly Phe Thr Val Ala
                245                 250                 255

Asn Glu Arg Phe Lys Lys Pro Thr Lys Asn Asp Leu Val Tyr Phe Glu
            260                 265                 270

Asn Ser Pro Asp Tyr Cys Ile Arg Asp Arg Glu Ala Gly Ser Leu Gly
        275                 280                 285

Thr Ala Gly Arg Val Cys Asn Leu Thr Ser Arg Gly Met Asp Ser Cys
    290                 295                 300

Glu Val Met Cys Cys Gly Arg Gly Tyr Asp Thr Ser His Val Thr Arg
305                 310                 315                 320

Met Thr Lys Cys Gly Cys Lys Phe His Trp Cys Cys Ala Val Arg Cys
                325                 330                 335

Gln Asp Cys Leu Glu Ala Leu Asp Val His Thr Cys Lys Ala Pro Lys
            340                 345                 350

Asn Ala Asp Trp Thr Thr Ala Thr
        355                 360

<210>  13
<211>  372
<212>  PRT
<213>  homo sapiens

<220>
<221>  misc_feature
<223>  Wnt 2B homo sapiens NP_004176 isoform 2B1

<400>  13

Met Leu Asp Gly Leu Gly Val Val Ala Ile Ser Ile Phe Gly Ile Gln
1               5                   10                  15

Leu Lys Thr Glu Gly Ser Leu Arg Thr Ala Val Pro Gly Ile Pro Thr
            20                  25                  30

Gln Ser Ala Phe Asn Lys Cys Leu Gln Arg Tyr Ile Gly Ala Leu Gly
        35                  40                  45

Ala Arg Val Ile Cys Asp Asn Ile Pro Gly Leu Val Ser Arg Gln Arg
    50                  55                  60

Gln Leu Cys Gln Arg Tyr Pro Asp Ile Met Arg Ser Val Gly Glu Gly
65                  70                  75                  80

Ala Arg Glu Trp Ile Arg Glu Cys Gln His Gln Phe Arg His His Arg
                85                  90                  95

Trp Asn Cys Thr Thr Leu Asp Arg Asp His Thr Val Phe Gly Arg Val
            100                 105                 110

Met Leu Arg Ser Ser Arg Glu Ala Ala Phe Val Tyr Ala Ile Ser Ser
        115                 120                 125

Ala Gly Val Val His Ala Ile Thr Arg Ala Cys Ser Gln Gly Glu Leu
    130                 135                 140

Ser Val Cys Ser Cys Asp Pro Tyr Thr Arg Gly Arg His His Asp Gln
145                 150                 155                 160

Arg Gly Asp Phe Asp Trp Gly Gly Cys Ser Asp Asn Ile His Tyr Gly
                165                 170                 175

Val Arg Phe Ala Lys Ala Phe Val Asp Ala Lys Glu Lys Arg Leu Lys
            180                 185                 190

Asp Ala Arg Ala Leu Met Asn Leu His Asn Asn Arg Cys Gly Arg Thr
        195                 200                 205

Ala Val Arg Arg Phe Leu Lys Leu Glu Cys Lys Cys His Gly Val Ser
    210                 215                 220

Gly Ser Cys Thr Leu Arg Thr Cys Trp Arg Ala Leu Ser Asp Phe Arg
225                 230                 235                 240

Arg Thr Gly Asp Tyr Leu Arg Arg Arg Tyr Asp Gly Ala Val Gln Val
                245                 250                 255

Met Ala Thr Gln Asp Gly Ala Asn Phe Thr Ala Ala Arg Gln Gly Tyr
            260                 265                 270

Arg Arg Ala Thr Arg Thr Asp Leu Val Tyr Phe Asp Asn Ser Pro Asp
        275                 280                 285

Tyr Cys Val Leu Asp Lys Ala Ala Gly Ser Leu Gly Thr Ala Gly Arg
    290                 295                 300

Val Cys Ser Lys Thr Ser Lys Gly Thr Asp Gly Cys Glu Ile Met Cys
305                 310                 315                 320

Cys Gly Arg Gly Tyr Asp Thr Thr Arg Val Thr Arg Val Thr Gln Cys
                325                 330                 335

Glu Cys Lys Phe His Trp Cys Cys Ala Val Arg Cys Lys Glu Cys Arg
            340                 345                 350

Asn Thr Val Asp Val His Thr Cys Lys Ala Pro Lys Lys Ala Glu Trp
        355                 360                 365

Leu Asp Gln Thr
    370

<210>  14
<211>  391
<212>  PRT
<213>  homo sapiens

<220>
<221>  misc_feature
<223>  Wnt 2B homo sapiens NP_078613 isoform 2B2

<400>  14

Met Leu Arg Pro Gly Gly Ala Glu Glu Ala Ala Gln Leu Pro Leu Arg
1               5                   10                  15

Arg Ala Ser Ala Pro Val Pro Val Pro Ser Pro Ala Ala Pro Asp Gly
            20                  25                  30

Ser Arg Ala Ser Ala Arg Leu Gly Leu Ala Cys Leu Leu Leu Leu Leu
        35                  40                  45

Leu Leu Thr Leu Pro Ala Arg Val Asp Thr Ser Trp Trp Tyr Ile Gly
    50                  55                  60

Ala Leu Gly Ala Arg Val Ile Cys Asp Asn Ile Pro Gly Leu Val Ser
65                  70                  75                  80

Arg Gln Arg Gln Leu Cys Gln Arg Tyr Pro Asp Ile Met Arg Ser Val
                85                  90                  95

Gly Glu Gly Ala Arg Glu Trp Ile Arg Glu Cys Gln His Gln Phe Arg
            100                 105                 110

His His Arg Trp Asn Cys Thr Thr Leu Asp Arg Asp His Thr Val Phe
        115                 120                 125

Gly Arg Val Met Leu Arg Ser Ser Arg Glu Ala Ala Phe Val Tyr Ala
    130                 135                 140

Ile Ser Ser Ala Gly Val Val His Ala Ile Thr Arg Ala Cys Ser Gln
145                 150                 155                 160

Gly Glu Leu Ser Val Cys Ser Cys Asp Pro Tyr Thr Arg Gly Arg His
                165                 170                 175

His Asp Gln Arg Gly Asp Phe Asp Trp Gly Gly Cys Ser Asp Asn Ile
            180                 185                 190

His Tyr Gly Val Arg Phe Ala Lys Ala Phe Val Asp Ala Lys Glu Lys
        195                 200                 205

Arg Leu Lys Asp Ala Arg Ala Leu Met Asn Leu His Asn Asn Arg Cys
    210                 215                 220

Gly Arg Thr Ala Val Arg Arg Phe Leu Lys Leu Glu Cys Lys Cys His
225                 230                 235                 240

Gly Val Ser Gly Ser Cys Thr Leu Arg Thr Cys Trp Arg Ala Leu Ser
                245                 250                 255

Asp Phe Arg Arg Thr Gly Asp Tyr Leu Arg Arg Arg Tyr Asp Gly Ala
            260                 265                 270

Val Gln Val Met Ala Thr Gln Asp Gly Ala Asn Phe Thr Ala Ala Arg
        275                 280                 285

Gln Gly Tyr Arg Arg Ala Thr Arg Thr Asp Leu Val Tyr Phe Asp Asn
    290                 295                 300

Ser Pro Asp Tyr Cys Val Leu Asp Lys Ala Ala Gly Ser Leu Gly Thr
305                 310                 315                 320

Ala Gly Arg Val Cys Ser Lys Thr Ser Lys Gly Thr Asp Gly Cys Glu
                325                 330                 335

Ile Met Cys Cys Gly Arg Gly Tyr Asp Thr Thr Arg Val Thr Arg Val

340                 345                 350

Thr Gln Cys Glu Cys Lys Phe His Trp Cys Cys Ala Val Arg Cys Lys
        355                 360                 365

Glu Cys Arg Asn Thr Val Asp Val His Thr Cys Lys Ala Pro Lys Lys
    370                 375                 380

Ala Glu Trp Leu Asp Gln Thr
385                 390

<210>  15
<211>  355
<212>  PRT
<213>  homo sapiens

<220>
<221>  misc_feature
<223>  Wnt 3 homo sapiens NP_110380

<400>  15

Met Glu Pro His Leu Leu Gly Leu Leu Leu Gly Leu Leu Leu Gly Gly
1               5                   10                  15

Thr Arg Val Leu Ala Gly Tyr Pro Ile Trp Trp Ser Leu Ala Leu Gly
            20                  25                  30

Gln Gln Tyr Thr Ser Leu Gly Ser Gln Pro Leu Leu Cys Gly Ser Ile
        35                  40                  45

Pro Gly Leu Val Pro Lys Gln Leu Arg Phe Cys Arg Asn Tyr Ile Glu
    50                  55                  60

Ile Met Pro Ser Val Ala Glu Gly Val Lys Leu Gly Ile Gln Glu Cys
65                  70                  75                  80

Gln His Gln Phe Arg Gly Arg Arg Trp Asn Cys Thr Thr Ile Asp Asp
                85                  90                  95

Ser Leu Ala Ile Phe Gly Pro Val Leu Asp Lys Ala Thr Arg Glu Ser
            100                 105                 110

Ala Phe Val His Ala Ile Ala Ser Ala Gly Val Ala Phe Ala Val Thr
        115                 120                 125

Arg Ser Cys Ala Glu Gly Thr Ser Thr Ile Cys Gly Cys Asp Ser His
    130                 135                 140

His Lys Gly Pro Pro Gly Glu Gly Trp Lys Trp Gly Gly Cys Ser Glu
145                 150                 155                 160

Asp Ala Asp Phe Gly Val Leu Val Ser Arg Glu Phe Ala Asp Ala Arg
                165                 170                 175

Glu Asn Arg Pro Asp Ala Arg Ser Ala Met Asn Lys His Asn Asn Glu
            180                 185                 190

Ala Gly Arg Thr Thr Ile Leu Asp His Met His Leu Lys Cys Lys Cys
        195                 200                 205

His Gly Leu Ser Gly Ser Cys Glu Val Lys Thr Cys Trp Trp Ala Gln
    210                 215                 220

Pro Asp Phe Arg Ala Ile Gly Asp Phe Leu Lys Asp Lys Tyr Asp Ser
225                 230                 235                 240

Ala Ser Glu Met Val Val Glu Lys His Arg Glu Ser Arg Gly Trp Val
                245                 250                 255

Glu Thr Leu Arg Ala Lys Tyr Ser Leu Phe Lys Pro Pro Thr Glu Arg
            260                 265                 270

Asp Leu Val Tyr Tyr Glu Asn Ser Pro Asn Phe Cys Glu Pro Asn Pro
        275                 280                 285

Glu Thr Gly Ser Phe Gly Thr Arg Asp Arg Thr Cys Asn Val Thr Ser
    290                 295                 300

His Gly Ile Asp Gly Cys Asp Leu Leu Cys Cys Gly Arg Gly His Asn
305                 310                 315                 320

Thr Arg Thr Glu Lys Arg Lys Glu Lys Cys His Cys Ile Phe His Trp
                325                 330                 335

Cys Cys Tyr Val Ser Cys Gln Glu Cys Ile Arg Ile Tyr Asp Val His
            340                 345                 350

Thr Cys Lys
        355

<210>  16
<211>  352
<212>  PRT

<213>  homo sapiens

<220>
<221>  misc_feature
<223>  Wnt3a homo sapiens NP_49122

<400>  16

Met Ala Pro Leu Gly Tyr Phe Leu Leu Leu Cys Ser Leu Lys Gln Ala
1               5                   10                  15

Leu Gly Ser Tyr Pro Ile Trp Trp Ser Leu Ala Val Gly Pro Gln Tyr
            20                  25                  30

Ser Ser Leu Gly Ser Gln Pro Ile Leu Cys Ala Ser Ile Pro Gly Leu
        35                  40                  45

Val Pro Lys Gln Leu Arg Phe Cys Arg Asn Tyr Val Glu Ile Met Pro
    50                  55                  60

Ser Val Ala Glu Gly Ile Lys Ile Gly Ile Gln Glu Cys Gln His Gln
65                  70                  75                  80

Phe Arg Gly Arg Arg Trp Asn Cys Thr Thr Val His Asp Ser Leu Ala
                85                  90                  95

Ile Phe Gly Pro Val Leu Asp Lys Ala Thr Arg Glu Ser Ala Phe Val
            100                 105                 110

His Ala Ile Ala Ser Ala Gly Val Ala Phe Ala Val Thr Arg Ser Cys
        115                 120                 125

Ala Glu Gly Thr Ala Ala Ile Cys Gly Cys Ser Ser Arg His Gln Gly
    130                 135                 140

Ser Pro Gly Lys Gly Trp Lys Trp Gly Gly Cys Ser Glu Asp Ile Glu
145                 150                 155                 160

Phe Gly Gly Met Val Ser Arg Glu Phe Ala Asp Ala Arg Glu Asn Arg
                165                 170                 175

Pro Asp Ala Arg Ser Ala Met Asn Arg His Asn Asn Glu Ala Gly Arg
            180                 185                 190

Gln Ala Ile Ala Ser His Met His Leu Lys Cys Lys Cys His Gly Leu
        195                 200                 205

Ser Gly Ser Cys Glu Val Lys Thr Cys Trp Trp Ser Gln Pro Asp Phe
    210                 215                 220

Arg Ala Ile Gly Asp Phe Leu Lys Asp Lys Tyr Asp Ser Ala Ser Glu
225                 230                 235                 240

Met Val Val Glu Lys His Arg Glu Ser Arg Gly Trp Val Glu Thr Leu
                245                 250                 255

Arg Pro Arg Tyr Thr Tyr Phe Lys Val Pro Thr Glu Arg Asp Leu Val
            260                 265                 270

Tyr Tyr Glu Ala Ser Pro Asn Phe Cys Glu Pro Asn Pro Glu Thr Gly
        275                 280                 285

Ser Phe Gly Thr Arg Asp Arg Thr Cys Asn Val Ser Ser His Gly Ile
    290                 295                 300

Asp Gly Cys Asp Leu Leu Cys Cys Gly Arg Gly His Asn Ala Arg Ala
305                 310                 315                 320

Glu Arg Arg Arg Glu Lys Cys Arg Cys Val Phe His Trp Cys Cys Tyr
                325                 330                 335

Val Ser Cys Gln Glu Cys Thr Arg Val Tyr Asp Val His Thr Cys Lys
            340                 345                 350

<210>  17
<211>  351
<212>  PRT
<213>  homo sapiens

<220>
<221>  misc_feature
<223>  Wnt4 homo sapiens NP_110388

<400>  17

Met Ser Pro Arg Ser Cys Leu Arg Ser Leu Arg Leu Leu Val Phe Ala
1               5                   10                  15

Val Phe Ser Ala Ala Ala Ser Asn Trp Leu Tyr Leu Ala Lys Leu Ser
            20                  25                  30

Ser Val Gly Ser Ile Ser Glu Glu Glu Thr Cys Glu Lys Leu Lys Gly
        35                  40                  45

Leu Ile Gln Arg Gln Val Gln Met Cys Lys Arg Asn Leu Glu Val Met

50                  55                  60

Asp Ser Val Arg Arg Gly Ala Gln Leu Ala Ile Glu Glu Cys Gln Tyr
65                  70                  75                  80

Gln Phe Arg Asn Arg Arg Trp Asn Cys Ser Thr Leu Asp Ser Leu Pro
                85                  90                  95

Val Phe Gly Lys Val Val Thr Gln Gly Thr Arg Glu Ala Ala Phe Val
            100                 105                 110

Tyr Ala Ile Ser Ser Ala Gly Val Ala Phe Ala Val Thr Arg Ala Cys
        115                 120                 125

Ser Ser Gly Glu Leu Glu Lys Cys Gly Cys Asp Arg Thr Val His Gly
    130                 135                 140

Val Ser Pro Gln Gly Phe Gln Trp Ser Gly Cys Ser Asp Asn Ile Ala
145                 150                 155                 160

Tyr Gly Val Ala Phe Ser Gln Ser Phe Val Asp Val Arg Glu Arg Ser
                165                 170                 175

Lys Gly Ala Ser Ser Ser Arg Ala Leu Met Asn Leu His Asn Asn Glu
            180                 185                 190

Ala Gly Arg Lys Ala Ile Leu Thr His Met Arg Val Glu Cys Lys Cys
        195                 200                 205

His Gly Val Ser Gly Ser Cys Glu Val Lys Thr Cys Trp Arg Ala Val
    210                 215                 220

Pro Pro Phe Arg Gln Val Gly His Ala Leu Lys Glu Lys Phe Asp Gly
225                 230                 235                 240

Ala Thr Glu Val Glu Pro Arg Arg Val Gly Ser Ser Arg Ala Leu Val
                245                 250                 255

Pro Arg Asn Ala Gln Phe Lys Pro His Thr Asp Glu Asp Leu Val Tyr
            260                 265                 270

Leu Glu Pro Ser Pro Asp Phe Cys Glu Gln Asp Met Arg Ser Gly Val
        275                 280                 285

Leu Gly Thr Arg Gly Arg Thr Cys Asn Lys Thr Ser Lys Ala Ile Asp
    290                 295                 300

Gly Cys Glu Leu Leu Cys Cys Gly Arg Gly Phe His Thr Ala Gln Val
305                 310                 315                 320

Glu Leu Ala Glu Arg Cys Ser Cys Lys Phe His Trp Cys Cys Phe Val
                325                 330                 335

Lys Cys Arg Gln Cys Gln Arg Leu Val Glu Leu His Thr Cys Arg
            340                 345                 350

<210>  18
<211>  380
<212>  PRT
<213>  homo sapiens

<220>
<221>  misc_feature
<223>  Wnt5a homo sapiens NP_003383

<400>  18

<210>  19
<211>  359
<212>  PRT
<213>  homo sapiens

<220>
<221>  misc_feature
<223>  Wnt 5b homo sapiens NP_110402

<400>  19

Met Pro Ser Leu Leu Leu Leu Phe Thr Ala Ala Leu Leu Ser Ser Trp
1               5                   10                  15

Ala Gln Leu Leu Thr Asp Ala Asn Ser Trp Trp Ser Leu Ala Leu Asn
            20                  25                  30

Pro Val Gln Arg Pro Glu Met Phe Ile Ile Gly Ala Gln Pro Val Cys
        35                  40                  45

Ser Gln Leu Pro Gly Leu Ser Pro Gly Gln Arg Lys Leu Cys Gln Leu
    50                  55                  60

Tyr Gln Glu His Met Ala Tyr Ile Gly Glu Gly Ala Lys Thr Gly Ile
65                  70                  75                  80

Lys Glu Cys Gln His Gln Phe Arg Gln Arg Arg Trp Asn Cys Ser Thr
                85                  90                  95

Ala Asp Asn Ala Ser Val Phe Gly Arg Val Met Gln Ile Gly Ser Arg
            100                 105                 110

Glu Thr Ala Phe Thr His Ala Val Ser Ala Ala Gly Val Val Asn Ala
        115                 120                 125

Ile Ser Arg Ala Cys Arg Glu Gly Glu Leu Ser Thr Cys Gly Cys Ser
    130                 135                 140

Arg Thr Ala Arg Pro Lys Asp Leu Pro Arg Asp Trp Leu Trp Gly Gly
145                 150                 155                 160

Cys Gly Asp Asn Val Glu Tyr Gly Tyr Arg Phe Ala Lys Glu Phe Val
                165                 170                 175

Asp Ala Arg Glu Arg Glu Lys Asn Phe Ala Lys Gly Ser Glu Glu Gln
            180                 185                 190

Gly Arg Val Leu Met Asn Leu Gln Asn Asn Glu Ala Gly Arg Arg Ala

195                 200                 205

Val Tyr Lys Met Ala Asp Val Ala Cys Lys Cys His Gly Val Ser Gly
    210                 215                 220

Ser Cys Ser Leu Lys Thr Cys Trp Leu Gln Leu Ala Glu Phe Arg Lys
225                 230                 235                 240

Val Gly Asp Arg Leu Lys Glu Lys Tyr Asp Ser Ala Ala Ala Met Arg
                245                 250                 255

Val Thr Arg Lys Gly Arg Leu Glu Leu Val Asn Ser Arg Phe Thr Gln
            260                 265                 270

Pro Thr Pro Glu Asp Leu Val Tyr Val Asp Pro Ser Pro Asp Tyr Cys
        275                 280                 285

Leu Arg Asn Glu Ser Thr Gly Ser Leu Gly Thr Gln Gly Arg Leu Cys
    290                 295                 300

Asn Lys Thr Ser Glu Gly Met Asp Gly Cys Glu Leu Met Cys Cys Gly
305                 310                 315                 320

Arg Gly Tyr Asn Gln Phe Lys Ser Val Gln Val Glu Arg Cys His Cys
                325                 330                 335

Lys Phe His Trp Cys Cys Phe Val Arg Cys Lys Lys Cys Thr Glu Ile
            340                 345                 350

Val Asp Gln Tyr Ile Cys Lys
        355

<210>  20
<211>  365
<212>  PRT
<213>  homo sapiens

<220>
<221>  misc_feature
<223>  Wnt6 homo sapiens NP_006513

<400>  20

Met Leu Pro Pro Leu Pro Ser Arg Leu Gly Leu Leu Leu Leu Leu Leu
1               5                   10                  15

Leu Cys Pro Ala His Val Gly Gly Leu Trp Trp Ala Val Gly Ser Pro
            20                  25                  30

Leu Val Met Asp Pro Thr Ser Ile Cys Arg Lys Ala Arg Arg Leu Ala
        35                  40                  45

Gly Arg Gln Ala Glu Leu Cys Gln Ala Glu Pro Glu Val Val Ala Glu
    50                  55                  60

Leu Ala Arg Gly Ala Arg Leu Gly Val Arg Glu Cys Gln Phe Gln Phe
65                  70                  75                  80

Arg Phe Arg Arg Trp Asn Cys Ser Ser His Ser Lys Ala Phe Gly Arg
                85                  90                  95

Ile Leu Gln Gln Asp Ile Arg Glu Thr Ala Phe Val Phe Ala Ile Thr
            100                 105                 110

Ala Ala Gly Ala Ser His Ala Val Thr Gln Ala Cys Ser Met Gly Glu
        115                 120                 125

Leu Leu Gln Cys Gly Cys Gln Ala Pro Arg Gly Arg Ala Pro Pro Arg
    130                 135                 140

Pro Ser Gly Leu Pro Gly Thr Pro Gly Pro Pro Gly Pro Ala Gly Ser
145                 150                 155                 160

Pro Glu Gly Ser Ala Ala Trp Glu Trp Gly Gly Cys Gly Asp Asp Val
                165                 170                 175

Asp Phe Gly Asp Glu Lys Ser Arg Leu Phe Met Asp Ala Arg His Lys
            180                 185                 190

Arg Gly Arg Gly Asp Ile Arg Ala Leu Val Gln Leu His Asn Asn Glu
        195                 200                 205

Ala Gly Arg Leu Ala Val Arg Ser His Thr Arg Thr Glu Cys Lys Cys
    210                 215                 220

His Gly Leu Ser Gly Ser Cys Ala Leu Arg Thr Cys Trp Gln Lys Leu
225                 230                 235                 240

Pro Pro Phe Arg Glu Val Gly Ala Arg Leu Leu Glu Arg Phe His Gly
                245                 250                 255

Ala Ser Arg Val Met Gly Thr Asn Asp Gly Lys Ala Leu Leu Pro Ala
            260                 265                 270

Val Arg Thr Leu Lys Pro Pro Gly Arg Ala Asp Leu Leu Tyr Ala Ala
        275                 280                 285

Asp Ser Pro Asp Phe Cys Ala Pro Asn Arg Arg Thr Gly Ser Pro Gly
    290                 295                 300

Thr Arg Gly Arg Ala Cys Asn Ser Ser Ala Pro Asp Leu Ser Gly Cys
305                 310                 315                 320

Asp Leu Leu Cys Cys Gly Arg Gly His Arg Gln Glu Ser Val Gln Leu
                325                 330                 335

Glu Glu Asn Cys Leu Cys Arg Phe His Trp Cys Cys Val Val Gln Cys
            340                 345                 350

His Arg Cys Arg Val Arg Lys Glu Leu Ser Leu Cys Leu
        355                 360                 365

<210>  21
<211>  349
<212>  PRT
<213>  homo sapiens

<220>
<221>  misc_feature
<223>  Wnt7a homo sapiens NP_004616

<400>  21

<210>  22
<211>  349
<212>  PRT
<213>  homo sapiens

<220>
<221>  misc_feature
<223>  Wnt7b, homo sapiens, NP_478679

<400>  22

Met His Arg Asn Phe Arg Lys Trp Ile Phe Tyr Val Phe Leu Cys Phe
1               5                   10                  15

Gly Val Leu Tyr Val Lys Leu Gly Ala Leu Ser Ser Val Val Ala Leu
            20                  25                  30

Gly Ala Asn Ile Ile Cys Asn Lys Ile Pro Gly Leu Ala Pro Arg Gln
        35                  40                  45

Arg Ala Ile Cys Gln Ser Arg Pro Asp Ala Ile Ile Val Ile Gly Glu
    50                  55                  60

Gly Ala Gln Met Gly Ile Asn Glu Cys Gln Tyr Gln Phe Arg Phe Gly
65                  70                  75                  80

Arg Trp Asn Cys Ser Ala Leu Gly Glu Lys Thr Val Phe Gly Gln Glu
                85                  90                  95

Leu Arg Val Gly Ser Arg Glu Ala Ala Phe Thr Tyr Ala Ile Thr Ala
            100                 105                 110

Ala Gly Val Ala His Ala Val Thr Ala Ala Cys Ser Gln Gly Asn Leu
        115                 120                 125

Ser Asn Cys Gly Cys Asp Arg Glu Lys Gln Gly Tyr Tyr Asn Gln Ala
    130                 135                 140

Glu Gly Trp Lys Trp Gly Gly Cys Ser Ala Asp Val Arg Tyr Gly Ile
145                 150                 155                 160

Asp Phe Ser Arg Arg Phe Val Asp Ala Arg Glu Ile Lys Lys Asn Ala
                165                 170                 175

Arg Arg Leu Met Asn Leu His Asn Asn Glu Ala Gly Arg Lys Val Leu
            180                 185                 190

Glu Asp Arg Met Gln Leu Glu Cys Lys Cys His Gly Val Ser Gly Ser
        195                 200                 205

Cys Thr Thr Lys Thr Cys Trp Thr Thr Leu Pro Lys Phe Arg Glu Val
    210                 215                 220

Gly His Leu Leu Lys Glu Lys Tyr Asn Ala Ala Val Gln Val Glu Val
225                 230                 235                 240

Val Arg Ala Ser Arg Leu Arg Gln Pro Thr Phe Leu Arg Ile Lys Gln
                245                 250                 255

Leu Arg Ser Tyr Gln Lys Pro Met Glu Thr Asp Leu Val Tyr Ile Glu
            260                 265                 270

Lys Ser Pro Asn Tyr Cys Glu Glu Asp Ala Ala Thr Gly Ser Val Gly
        275                 280                 285

Thr Gln Gly Arg Leu Cys Asn Arg Thr Ser Pro Gly Ala Asp Gly Cys
    290                 295                 300

Asp Thr Met Cys Cys Gly Arg Gly Tyr Asn Thr His Gln Tyr Thr Lys
305                 310                 315                 320

Val Trp Gln Cys Asn Cys Lys Phe His Trp Cys Cys Phe Val Lys Cys
                325                 330                 335

Asn Thr Cys Ser Glu Arg Thr Glu Val Phe Thr Cys Lys
            340                 345

<210>  23
<211>  351
<212>  PRT
<213>  homo sapiens

<220>
<221>  misc_feature
<223>  Wnt8a homo sapiens NP_490645

<400>  23

Met Gly Asn Leu Phe Met Leu Trp Ala Ala Leu Gly Ile Cys Cys Ala
1               5                   10                  15

Ala Phe Ser Ala Ser Ala Trp Ser Val Asn Asn Phe Leu Ile Thr Gly
            20                  25                  30

Pro Lys Ala Tyr Leu Thr Tyr Thr Thr Ser Val Ala Leu Gly Ala Gln
        35                  40                  45

Ser Gly Ile Glu Glu Cys Lys Phe Gln Phe Ala Trp Glu Arg Trp Asn
    50                  55                  60

Cys Pro Glu Asn Ala Leu Gln Leu Ser Thr His Asn Arg Leu Arg Ser
65                  70                  75                  80

Ala Thr Arg Glu Thr Ser Phe Ile His Ala Ile Ser Ser Ala Gly Val
                85                  90                  95

Met Tyr Ile Ile Thr Lys Asn Cys Ser Met Gly Asp Phe Glu Asn Cys
            100                 105                 110

Gly Cys Asp Gly Ser Asn Asn Gly Lys Thr Gly Gly His Gly Trp Ile
        115                 120                 125

Trp Gly Gly Cys Ser Asp Asn Val Glu Phe Gly Glu Arg Ile Ser Lys
    130                 135                 140

Leu Phe Val Asp Ser Leu Glu Lys Gly Lys Asp Ala Arg Ala Leu Met
145                 150                 155                 160

Asn Leu His Asn Asn Arg Ala Gly Arg Leu Ala Val Arg Ala Thr Met
                165                 170                 175

Lys Arg Thr Cys Lys Cys His Gly Ile Ser Gly Ser Cys Ser Ile Gln
            180                 185                 190

Thr Cys Trp Leu Gln Leu Ala Glu Phe Arg Glu Met Gly Asp Tyr Leu
        195                 200                 205

Lys Ala Lys Tyr Asp Gln Ala Leu Lys Ile Glu Met Asp Lys Arg Gln
    210                 215                 220

Leu Arg Ala Gly Asn Ser Ala Glu Gly His Trp Val Pro Ala Glu Ala
225                 230                 235                 240

Phe Leu Pro Ser Ala Glu Ala Glu Leu Ile Phe Leu Glu Glu Ser Pro
                245                 250                 255

Asp Tyr Cys Thr Cys Asn Ser Ser Leu Gly Ile Tyr Gly Thr Glu Gly
            260                 265                 270

Arg Glu Cys Leu Gln Asn Ser His Asn Thr Ser Arg Trp Glu Arg Arg
        275                 280                 285

Ser Cys Gly Arg Leu Cys Thr Glu Cys Gly Leu Gln Val Glu Glu Arg
    290                 295                 300

Lys Thr Glu Val Ile Ser Ser Cys Asn Cys Lys Phe Gln Trp Cys Cys
305                 310                 315                 320

Thr Val Lys Cys Asp Gln Cys Arg His Val Val Ser Lys Tyr Tyr Cys
                325                 330                 335

Ala Arg Ser Pro Gly Ser Ala Gln Ser Leu Gly Lys Gly Ser Ala
            340                 345                 350

<210>  24
<211>  351
<212>  PRT
<213>  homo sapiens

<220>
<221>  misc_feature
<223>  Wnt8b homo sapiens  NP_003384

<400>  24

Met Phe Leu Ser Lys Pro Ser Val Tyr Ile Cys Leu Phe Thr Cys Val
1               5                   10                  15

Leu Gln Leu Ser His Ser Trp Ser Val Asn Asn Phe Leu Met Thr Gly
            20                  25                  30

Pro Lys Ala Tyr Leu Ile Tyr Ser Ser Ser Val Ala Ala Gly Ala Gln
        35                  40                  45

Ser Gly Ile Glu Glu Cys Lys Tyr Gln Phe Ala Trp Asp Arg Trp Asn
    50                  55                  60

Cys Pro Glu Arg Ala Leu Gln Leu Ser Ser His Gly Gly Leu Arg Ser
65                  70                  75                  80

Ala Asn Arg Glu Thr Ala Phe Val His Ala Ile Ser Ser Ala Gly Val
                85                  90                  95

Met Tyr Thr Leu Thr Arg Asn Cys Ser Leu Gly Asp Phe Asp Asn Cys
            100                 105                 110

Gly Cys Asp Asp Ser Arg Asn Gly Gln Leu Gly Gly Gln Gly Trp Leu

115                 120                 125

Trp Gly Gly Cys Ser Asp Asn Val Gly Phe Gly Glu Ala Ile Ser Lys
    130                 135                 140

Gln Phe Val Asp Ala Leu Glu Thr Gly Gln Asp Ala Arg Ala Ala Met
145                 150                 155                 160

Asn Leu His Asn Asn Glu Ala Gly Arg Lys Ala Val Lys Gly Thr Met
                165                 170                 175

Lys Arg Thr Cys Lys Cys His Gly Val Ser Gly Ser Cys Thr Thr Gln
            180                 185                 190

Thr Cys Trp Leu Gln Leu Pro Glu Phe Arg Glu Val Gly Ala His Leu
        195                 200                 205

Lys Glu Lys Tyr His Ala Ala Leu Lys Val Asp Leu Leu Gln Gly Ala
    210                 215                 220

Gly Asn Ser Ala Ala Gly Arg Gly Ala Ile Ala Asp Thr Phe Arg Ser
225                 230                 235                 240

Ile Ser Thr Arg Glu Leu Val His Leu Glu Asp Ser Pro Asp Tyr Cys
                245                 250                 255

Leu Glu Asn Lys Thr Leu Gly Leu Leu Gly Thr Glu Gly Arg Glu Cys
            260                 265                 270

Leu Arg Arg Gly Arg Ala Leu Gly Arg Trp Glu Arg Arg Ser Cys Arg
        275                 280                 285

Arg Leu Cys Gly Asp Cys Gly Leu Ala Val Glu Glu Arg Arg Ala Glu
    290                 295                 300

Thr Val Ser Ser Cys Asn Cys Lys Phe His Trp Cys Cys Ala Val Arg
305                 310                 315                 320

Cys Glu Gln Cys Arg Arg Arg Val Thr Lys Tyr Phe Cys Ser Arg Ala
                325                 330                 335

Glu Arg Pro Arg Gly Gly Ala Ala His Lys Pro Gly Arg Lys Pro
            340                 345                 350

<210>  25
<211>  365

<212>  PRT
<213>  homo sapiens

<220>
<221>  misc_feature
<223>  Wnt9a homo sapiens NP_003386

<400>  25

Met Leu Asp Gly Ser Pro Leu Ala Arg Trp Leu Ala Ala Ala Phe Gly
1               5                   10                  15

Leu Thr Leu Leu Leu Ala Ala Leu Arg Pro Ser Ala Ala Tyr Phe Gly
            20                  25                  30

Leu Thr Gly Ser Glu Pro Leu Thr Ile Leu Pro Leu Thr Leu Glu Pro
        35                  40                  45

Glu Ala Ala Ala Gln Ala His Tyr Lys Ala Cys Asp Arg Leu Lys Leu
    50                  55                  60

Glu Arg Lys Gln Arg Arg Met Cys Arg Arg Asp Pro Gly Val Ala Glu
65                  70                  75                  80

Thr Leu Val Glu Ala Val Ser Met Ser Ala Leu Glu Cys Gln Phe Gln
                85                  90                  95

Phe Arg Phe Glu Arg Trp Asn Cys Thr Leu Glu Gly Arg Tyr Arg Ala
            100                 105                 110

Ser Leu Leu Lys Arg Gly Phe Lys Glu Thr Ala Phe Leu Tyr Ala Ile
        115                 120                 125

Ser Ser Ala Gly Leu Thr His Ala Leu Ala Lys Ala Cys Ser Ala Gly
    130                 135                 140

Arg Met Glu Arg Cys Thr Cys Asp Glu Ala Pro Asp Leu Glu Asn Arg
145                 150                 155                 160

Glu Ala Trp Gln Trp Gly Gly Cys Gly Asp Asn Leu Lys Tyr Ser Ser
                165                 170                 175

Lys Phe Val Lys Glu Phe Leu Gly Arg Arg Ser Ser Lys Asp Leu Arg
            180                 185                 190

Ala Arg Val Asp Phe His Asn Asn Leu Val Gly Val Lys Val Ile Lys
        195                 200                 205

Ala Gly Val Glu Thr Thr Cys Lys Cys His Gly Val Ser Gly Ser Cys
    210                 215                 220

Thr Val Arg Thr Cys Trp Arg Gln Leu Ala Pro Phe His Glu Val Gly
225                 230                 235                 240

Lys His Leu Lys His Lys Tyr Glu Thr Ala Leu Lys Val Gly Ser Thr
                245                 250                 255

Thr Asn Glu Ala Ala Gly Glu Ala Gly Ala Ile Ser Pro Pro Arg Gly
            260                 265                 270

Arg Ala Ser Gly Ala Gly Gly Ser Asp Pro Leu Pro Arg Thr Pro Glu
        275                 280                 285

Leu Val His Leu Asp Asp Ser Pro Ser Phe Cys Leu Ala Gly Arg Phe
    290                 295                 300

Ser Pro Gly Thr Ala Gly Arg Arg Cys His Arg Glu Lys Asn Cys Glu
305                 310                 315                 320

Ser Ile Cys Cys Gly Arg Gly His Asn Thr Gln Ser Arg Val Val Thr
                325                 330                 335

Arg Pro Cys Gln Cys Gln Val Arg Trp Cys Cys Tyr Val Glu Cys Arg
            340                 345                 350

Gln Cys Thr Gln Arg Glu Glu Val Tyr Thr Cys Lys Gly
        355                 360                 365

<210>  26
<211>  357
<212>  PRT
<213>  homo sapiens

<220>
<221>  misc_feature
<223>  Wnt9b homo sapiens NP_003387

<400>  26

Met Arg Pro Pro Pro Ala Leu Ala Leu Ala Gly Leu Cys Leu Leu Ala
1               5                   10                  15

Leu Pro Ala Ala Ala Ala Ser Tyr Phe Gly Leu Thr Gly Arg Glu Val
            20                  25                  30

Leu Thr Pro Phe Pro Gly Leu Gly Thr Ala Ala Ala Pro Ala Gln Gly
        35                  40                  45

Gly Ala His Leu Lys Gln Cys Asp Leu Leu Lys Leu Ser Arg Arg Gln
    50                  55                  60

Lys Gln Leu Cys Arg Arg Glu Pro Gly Leu Ala Glu Thr Leu Arg Asp
65                  70                  75                  80

Ala Ala His Leu Gly Leu Leu Glu Cys Gln Phe Gln Phe Arg His Glu
                85                  90                  95

Arg Trp Asn Cys Ser Leu Glu Gly Arg Met Gly Leu Leu Lys Arg Gly
            100                 105                 110

Phe Lys Glu Thr Ala Phe Leu Tyr Ala Val Ser Ser Ala Ala Leu Thr
        115                 120                 125

His Thr Leu Ala Arg Ala Cys Ser Ala Gly Arg Met Glu Arg Cys Thr
    130                 135                 140

Cys Asp Asp Ser Pro Gly Leu Glu Ser Arg Gln Ala Trp Gln Trp Gly
145                 150                 155                 160

Val Cys Gly Asp Asn Leu Lys Tyr Ser Thr Lys Phe Leu Ser Asn Phe
                165                 170                 175

Leu Gly Ser Lys Arg Gly Asn Lys Asp Leu Arg Ala Arg Ala Asp Ala
            180                 185                 190

His Asn Thr His Val Gly Ile Lys Ala Val Lys Ser Gly Leu Arg Thr
        195                 200                 205

Thr Cys Lys Cys His Gly Val Ser Gly Ser Cys Ala Val Arg Thr Cys
    210                 215                 220

Trp Lys Gln Leu Ser Pro Phe Arg Glu Thr Gly Gln Val Leu Lys Leu
225                 230                 235                 240

Arg Tyr Asp Ser Ala Val Lys Val Ser Ser Ala Thr Asn Glu Ala Leu
                245                 250                 255

Gly Arg Leu Glu Leu Trp Ala Pro Ala Arg Gln Gly Ser Leu Thr Lys
            260                 265                 270

Gly Leu Ala Pro Arg Ser Gly Asp Leu Val Tyr Met Glu Asp Ser Pro

275                 280                 285

Ser Phe Cys Arg Pro Ser Lys Tyr Ser Pro Gly Thr Ala Gly Arg Val
    290                 295                 300

Cys Ser Arg Glu Ala Ser Cys Ser Ser Leu Cys Cys Gly Arg Gly Tyr
305                 310                 315                 320

Asp Thr Gln Ser Arg Leu Val Ala Phe Ser Cys His Cys Gln Val Gln
                325                 330                 335

Trp Cys Cys Tyr Val Glu Cys Gln Gln Cys Val Gln Glu Glu Leu Val
            340                 345                 350

Tyr Thr Cys Lys His
        355

<210>  27
<211>  417
<212>  PRT
<213>  homo sapiens

<220>
<221>  misc_feature
<223>  Wnt10a homo sapiens NP_079492

<400>  27

<210>  28
<211>  389
<212>  PRT
<213>  homo sapiens

<220>
<221>  misc_feature
<223>  Wnt10b homo sapiens NP_003385

<400>  28

<210>  29
<211>  354
<212>  PRT
<213>  homo sapiens

<220>
<221>  misc_feature
<223>  Wnt11 homo sapiens NP_004617

<400>  29

Met Arg Ala Arg Pro Gln Val Cys Glu Ala Leu Leu Phe Ala Leu Ala
1               5                   10                  15

Leu Gln Thr Gly Val Cys Tyr Gly Ile Lys Trp Leu Ala Leu Ser Lys
            20                  25                  30

Thr Pro Ser Ala Leu Ala Leu Asn Gln Thr Gln His Cys Lys Gln Leu
        35                  40                  45

Glu Gly Leu Val Ser Ala Gln Val Gln Leu Cys Arg Ser Asn Leu Glu
    50                  55                  60

Leu Met His Thr Val Val His Ala Ala Arg Glu Val Met Lys Ala Cys
65                  70                  75                  80

Arg Arg Ala Phe Ala Asp Met Arg Trp Asn Cys Ser Ser Ile Glu Leu
                85                  90                  95

Ala Pro Asn Tyr Leu Leu Asp Leu Glu Arg Gly Thr Arg Glu Ser Ala
            100                 105                 110

Phe Val Tyr Ala Leu Ser Ala Ala Ala Ile Ser His Ala Ile Ala Arg
        115                 120                 125

Ala Cys Thr Ser Gly Asp Leu Pro Gly Cys Ser Cys Gly Pro Val Pro
    130                 135                 140

Gly Glu Pro Pro Gly Pro Gly Asn Arg Trp Gly Gly Cys Ala Asp Asn
145                 150                 155                 160

Leu Ser Tyr Gly Leu Leu Met Gly Ala Lys Phe Ser Asp Ala Pro Met
                165                 170                 175

Lys Val Lys Lys Thr Gly Ser Gln Ala Asn Lys Leu Met Arg Leu His
            180                 185                 190

Asn Ser Glu Val Gly Arg Gln Ala Leu Arg Ala Ser Leu Glu Met Lys
        195                 200                 205

Cys Lys Cys His Gly Val Ser Gly Ser Cys Ser Ile Arg Thr Cys Trp
    210                 215                 220

Lys Gly Leu Gln Glu Leu Gln Asp Val Ala Ala Asp Leu Lys Thr Arg
225                 230                 235                 240

Tyr Leu Ser Ala Thr Lys Val Val His Arg Pro Met Gly Thr Arg Lys
                245                 250                 255

His Leu Val Pro Lys Asp Leu Asp Ile Arg Pro Val Lys Asp Ser Glu
            260                 265                 270

Leu Val Tyr Leu Gln Ser Ser Pro Asp Phe Cys Met Lys Asn Glu Lys
        275                 280                 285

Val Gly Ser His Gly Thr Gln Asp Arg Gln Cys Asn Lys Thr Ser Asn
    290                 295                 300

Gly Ser Asp Ser Cys Asp Leu Met Cys Cys Gly Arg Gly Tyr Asn Pro
305                 310                 315                 320

Tyr Thr Asp Arg Val Val Glu Arg Cys His Cys Lys Tyr His Trp Cys
                325                 330                 335

Cys Tyr Val Thr Cys Arg Arg Cys Glu Arg Thr Val Glu Arg Tyr Val
            340                 345                 350

Cys Lys

<210>  30
<211>  355
<212>  PRT
<213>  homo sapiens

<220>

<221>  misc_feature
<223>  Wnt16 homo sapiens NP_057171 isoform2

<400>  30

Met Gln Leu Thr Thr Cys Leu Arg Glu Thr Leu Phe Thr Gly Ala Ser
1               5                   10                  15

Gln Lys Thr Ser Leu Trp Trp Leu Gly Ile Ala Ser Phe Gly Val Pro
            20                  25                  30

Glu Lys Leu Gly Cys Ala Asn Leu Pro Leu Asn Ser Arg Gln Lys Glu
        35                  40                  45

Leu Cys Lys Arg Lys Pro Tyr Leu Leu Pro Ser Ile Arg Glu Gly Ala
    50                  55                  60

Arg Leu Gly Ile Gln Glu Cys Gly Ser Gln Phe Arg His Glu Arg Trp
65                  70                  75                  80

Asn Cys Met Ile Thr Ala Ala Ala Thr Thr Ala Pro Met Gly Ala Ser
                85                  90                  95

Pro Leu Phe Gly Tyr Glu Leu Ser Ser Gly Thr Lys Glu Thr Ala Phe
            100                 105                 110

Ile Tyr Ala Val Met Ala Ala Gly Leu Val His Ser Val Thr Arg Ser
        115                 120                 125

Cys Ser Ala Gly Asn Met Thr Glu Cys Ser Cys Asp Thr Thr Leu Gln
    130                 135                 140

Asn Gly Gly Ser Ala Ser Glu Gly Trp His Trp Gly Gly Cys Ser Asp
145                 150                 155                 160

Asp Val Gln Tyr Gly Met Trp Phe Ser Arg Lys Phe Leu Asp Phe Pro
                165                 170                 175

Ile Gly Asn Thr Thr Gly Lys Glu Asn Lys Val Leu Leu Ala Met Asn
            180                 185                 190

Leu His Asn Asn Glu Ala Gly Arg Gln Ala Val Ala Lys Leu Met Ser
        195                 200                 205

Val Asp Cys Arg Cys His Gly Val Ser Gly Ser Cys Ala Val Lys Thr
    210                 215                 220

Cys Trp Lys Thr Met Ser Ser Phe Glu Lys Ile Gly His Leu Leu Lys
225                 230                 235                 240

Asp Lys Tyr Glu Asn Ser Ile Gln Ile Ser Asp Lys Thr Lys Arg Lys
                245                 250                 255

Met Arg Arg Arg Glu Lys Asp Gln Arg Lys Ile Pro Ile His Lys Asp
            260                 265                 270

Asp Leu Leu Tyr Val Asn Lys Ser Pro Asn Tyr Cys Val Glu Asp Lys
        275                 280                 285

Lys Leu Gly Ile Pro Gly Thr Gln Gly Arg Glu Cys Asn Arg Thr Ser
    290                 295                 300

Glu Gly Ala Asp Gly Cys Asn Leu Leu Cys Cys Gly Arg Gly Tyr Asn
305                 310                 315                 320

Thr His Val Val Arg His Val Glu Arg Cys Glu Cys Lys Phe Ile Trp
                325                 330                 335

Cys Cys Tyr Val Arg Cys Arg Arg Cys Glu Ser Met Thr Asp Val His
            340                 345                 350

Thr Cys Lys
        355

<210>  31
<211>  365
<212>  PRT
<213>  homo sapiens

<220>
<221>  misc_feature
<223>  Wnt16 homo sapiens NP_476509 isoform 1

<400>  31

Met Asp Arg Ala Ala Leu Leu Gly Leu Ala Arg Leu Cys Ala Leu Trp
1               5                   10                  15

Ala Ala Leu Leu Val Leu Phe Pro Tyr Gly Ala Gln Gly Asn Trp Met
            20                  25                  30

Trp Leu Gly Ile Ala Ser Phe Gly Val Pro Glu Lys Leu Gly Cys Ala
        35                  40                  45

Asn Leu Pro Leu Asn Ser Arg Gln Lys Glu Leu Cys Lys Arg Lys Pro

50                  55                  60

Tyr Leu Leu Pro Ser Ile Arg Glu Gly Ala Arg Leu Gly Ile Gln Glu
65                  70                  75                  80

Cys Gly Ser Gln Phe Arg His Glu Arg Trp Asn Cys Met Ile Thr Ala
                85                  90                  95

Ala Ala Thr Thr Ala Pro Met Gly Ala Ser Pro Leu Phe Gly Tyr Glu
            100                 105                 110

Leu Ser Ser Gly Thr Lys Glu Thr Ala Phe Ile Tyr Ala Val Met Ala
        115                 120                 125

Ala Gly Leu Val His Ser Val Thr Arg Ser Cys Ser Ala Gly Asn Met
    130                 135                 140

Thr Glu Cys Ser Cys Asp Thr Thr Leu Gln Asn Gly Gly Ser Ala Ser
145                 150                 155                 160

Glu Gly Trp His Trp Gly Gly Cys Ser Asp Asp Val Gln Tyr Gly Met
                165                 170                 175

Trp Phe Ser Arg Lys Phe Leu Asp Phe Pro Ile Gly Asn Thr Thr Gly
            180                 185                 190

Lys Glu Asn Lys Val Leu Leu Ala Met Asn Leu His Asn Asn Glu Ala
        195                 200                 205

Gly Arg Gln Ala Val Ala Lys Leu Met Ser Val Asp Cys Arg Cys His
    210                 215                 220

Gly Val Ser Gly Ser Cys Ala Val Lys Thr Cys Trp Lys Thr Met Ser
225                 230                 235                 240

Ser Phe Glu Lys Ile Gly His Leu Leu Lys Asp Lys Tyr Glu Asn Ser
                245                 250                 255

Ile Gln Ile Ser Asp Lys Thr Lys Arg Lys Met Arg Arg Arg Glu Lys
            260                 265                 270

Asp Gln Arg Lys Ile Pro Ile His Lys Asp Asp Leu Leu Tyr Val Asn
        275                 280                 285

Lys Ser Pro Asn Tyr Cys Val Glu Asp Lys Lys Leu Gly Ile Pro Gly
    290                 295                 300

Thr Gln Gly Arg Glu Cys Asn Arg Thr Ser Glu Gly Ala Asp Gly Cys
305                 310                 315                 320

Asn Leu Leu Cys Cys Gly Arg Gly Tyr Asn Thr His Val Val Arg His
                325                 330                 335

Val Glu Arg Cys Glu Cys Lys Phe Ile Trp Cys Cys Tyr Val Arg Cys
            340                 345                 350

Arg Arg Cys Glu Ser Met Thr Asp Val His Thr Cys Lys
        355                 360                 365